# EUROPEAN PATENT SPECIFICATION

(11) **EP 0 764 210 B1**
(45) Date of publication and mention of the grant of the patent: **11.01.2006**
(21) Application number: 95918126.4
(22) Date of filing: 30.05.1995
(51) Int. Cl.: C12N 15/32, C07K 14/325, C12N 15/82, A01H 5/00, A01N 63/00, C12N 1/21, C12N 1/19, C12R 1/07, C12R 1/38, C12R 1/41, C12R 1/01

(54) **NOVEL BACILLUS THURINGIENSIS GENES CODING TOXINS ACTIVE AGAINST LEPIDOPTERAN PESTS**
NEUE BACILLUS THURINGIENSIS GENE KODIEREND FÜR TOXINE DIE GEGEN LEPIDOPSTERE-SCHÄDLINGE AKTIV SIND
NOUVEAUX GENES DU BACILLUS THURINGIENSIS CODANT POUR DES TOXINES ACTIVES CONTRE LES LEPIDOPTERES

(30) Priority: 10.06.1994 US 257999
(43) Date of publication of application: 26.03.1997
(73) Proprietor: Syngenta Participations AG, 4058 Basel (CH)
(72) Inventor: KRAMER, Vance, Cary, Hillsborough, NC 27278 (US); CURRIER, Thomas, Curtis, Chapel Hill, NC 27514 (US)
(74) Representative: Gaal, Jozsef Christopher
(86) International application number: PCT/IB1995/000413
(87) International publication number: WO 1995/034656

(56) References cited:
- WO-A-93/09218
- US-A- 5 296 368
- JOURNAL OF BACTERIOLOGY, vol. 172, no. 12, December 1990 pages 6783-6788, VISSER, B. ET AL. 'A novel Bacillus thuringiensis gene encoding a Spodoptera exigua-specific crystal protein'
- BIOTECHNOLOGY, vol. 11, February 1993 NEW YORK US, pages 194-200, KOZIEL, M. ET AL. 'Field performance of elite transgenic maize plants expressing an insecticidal protein derived from Bacillus thuringiensis' cited in the application
- ENTWISTLE, P. et al.'Bacillus thuringiensis, an environmental biopesticide: theory and practice'; 1993, JOHN WILEY & Sons Ltd.
- MOAR W.J. ET AL: 'Insecticidal activity of the CryIIA protein from the NRD-12 isolate of Bacillus thurigiensis subsp. kurstaki expressed in Escherichia coli and Bacillus thurigiensis and in a leaf-colonizing strain of Bacillus cereus' APPLIED AND ENVIRONMENTAL MICROBIOLOGY vol. 60, no. 3, March 1994, pages 896 - 902
- DE MAAGD R. ET AL: 'How Bacillus thurigiensis has evolved specific toxins to colonize the insect world' TRENDS IN GENETICS vol. 17, no. 4, April 2001, pages 193 - 199
- VAN FRANKENHUYZEN K. ET AL: 'Toxicity of activated CryI proteins from Bacillus thuringiensis to six forest lepidoptera and bombyx mori' JOURNAL OF INVERTEBRATE PATHOLOGY vol. 62, 1993, pages 295 - 301
- HONéE G. ET AL: 'The C-terminal domain of the toxic fragment of a Bacillus thurigiensis crystal protein determines receptor binding' MOLECULAR MICROBIOLOGY vol. 5, no. 11, 1991, pages 2799 - 2806, XP000567679

### SEQUENCE LISTING

(1) GENERAL INFORMATION:
   (i) APPLICANT:
      (A) NAME: CIBA-GEIGY AG
      (B) STREET: Klybeckstr. 141
      (C) CITY: Basel
      (E) COUNTRY: Switzerland
      (F) POSTAL CODE (ZIP): 4002
      (G) TELEPHONE: +41 61 69 11 11
      (H) TELEFAX: + 41 61 696 79 76
      (I) TELEX: 962 991
   (ii) TITLE OF INVENTION: NOVEL *BACILLUS THURINGIENSIS* GENE ENCODING A TOXIN ACTIVE AGAINST INSECTS
   (iii) NUMBER OF SEQUENCES: 18
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25 (EPO)
(2) INFORMATION FOR SEQ ID NO:1:
   (i) SEQUENCE CHARACTERISTICS:
      (A) LENGTH: 4003 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (iv) ANTI-SENSE: NO
   (vi) ORIGINAL SOURCE:
      (A) ORGANISM: Bacillus thuringiensis
      (B) STRAIN: kurstaki
      (C) INDIVIDUAL ISOLATE: CGB316
   (ix) FEATURE:
      (A) NAME/KEY: promoter
      (B) LOCATION: 96..124
      (D) OTHER INFORMATION: /function= "putative promoter region"
   (ix) FEATURE:
      (A) NAME/KEY: RBS
      (B) LOCATION: 185..190
   (ix) FEATURE:
      (A) NAME/KEY: CDS
      (B) LOCATION: 196..3723
      (D) OTHER INFORMATION: /product= "Full-length CryIE(c) protein"
   (ix) FEATURE:
      (A) NAME/KEY: misc_feature
      (B) LOCATION: 1191..1590
      (D) OTHER INFORMATION: /note= "This region of the CryIE(c) DNA sequence encodes the amino acid sequence designated Sub-Sequence A"
   (ix) FEATURE:
      (A) NAME/KEY: misc_feature
      (B) LOCATION: 1591..2061
      (D) OTHER INFORMATION: /note= "This region of the CryIE(c) DNA sequence encodes the amino acid sequence designated Sub-Sequence B"
   (xi) SEQUENCE DESCRIPTION: SEQ ID NO:1:
   AAGTATGGGC GAGAAATAAG TGAATTATTA GCACCCTGGG TCTATTTTAG CCCCAGGGCA      60
   TAAATTGATA TTTTTTAATA TTGGTTGCAC TTTGTGTATT TTTTCATAGA ATGATTCATA     120
   TACTTTACAT TGTAATACGA TACGAGGTTT TTAGTTTTAA AGAACAACTA TTATGATGAA     180
   AAACGGAGGG TTATA ATG GAT AAT AAT AAT CAA AAT CAA TGC ATA CCT TAC     231
                    Met Asp Asn Asn Asn Gln Asn Gln Cys Ile Pro Tyr
                     1               5                  10
   AAT TGT TTA AGT AAT CCT GAA TTA GAG ATA TTA GAG ATT GAA AGA TCA      279
   Asn Cys Leu Ser Asn Pro Glu Leu Glu Ile Leu Glu Ile Glu Arg Ser
            15                  20                  25
   AAT AAT ACC GTA GTA GAA GAC ATC ACC TTG GGG CTG AGT CGT CTG CTC      327
   Asn Asn Thr Val Val Glu Asp Ile Thr Leu Gly Leu Ser Arg Leu Leu
        30                  35                  40
   GTT TCC GCA ATT CCA CTA GGG GAT TTT ATA CTT GGC TTG TTT GAT GTA      375
   Val Ser Ala Ile Pro Leu Gly Asp Phe Ile Leu Gly Leu Phe Asp Val
    45                  50                  55                  60
   ATA TGG GGG GCT TTA GGT CGT TCA GAG TGG GAT ATA TTT TTA GAG CAA      423
   Ile Trp Gly Ala Leu Gly Arg Ser Glu Trp Asp Ile Phe Leu Glu Gln
                    65                  70                  75
   ATT GAG CTA TTG ATC GGC CAA AGA ATA GAG GAA TTT GCT AGG AAT CAG 471
   Ile Glu Leu Leu Ile Gly Gln Arg Ile Glu Glu Phe Ala Arg Asn Gln
   80 85 90
   GCA ATT TCT AGA TTA GAA GGG CTA AGC AAT CTT TAT CGA ATT TAC ACA 519
   Ala Ile Ser Arg Leu Glu Gly Leu Ser Asn Leu Tyr Arg Ile Tyr Thr
   95 100 105
   AAT GCT TTT AAA GAC TGG GAA GCA GAT CCT ACT AAT CTA GAA TTA AAA 567
   Asn Ala Phe Lys Asp Trp Glu Ala Asp Pro Thr Asn Leu Glu Leu Lys
   110 115 120
   GAA GAG ATG CGT ACT CAA TTT AAT GAC ATG AAC AGT GCT TTT ACA ACA 615
   Glu Glu Met Arg Thr Gln Phe Asn Asp Met Asn Ser Ala Phe Thr Thr
   125 130 135 140
   GCT ATT CCT CTT TTT TCA GTT CGA GGT TAT GAA CTT CCT CTT TTA TCA 663
   Ala Ile Pro Leu Phe Ser Val Arg Gly Tyr Glu Leu Pro Leu Leu Ser
   145 150 155
   GTA TAT GTT CAA GCT GCA AAT TTA CAT TTA TCA GTT TTA AGA GAT GTT 711
   Val Tyr Val Gln Ala Ala Asn Leu His Leu Ser Val Leu Arg Asp Val
   160 165 170
   TCA GTG TTT GGA CAA CGT TGG GGA TTT GAC GTA GCA ACA GTC AAT CGT 759
   Ser Val Phe Gly Gln Arg Trp Gly Phe Asp Val Ala Thr Val Asn Arg
   175 180 185
   CGT TAT GAT GAT TTA ACT ACG AAT ATA GGC GAC TAC ACA GAT TAT GCT 807
   Arg Tyr Asp Asp Leu Thr Thr Asn Ile Gly Asp Tyr Thr Asp Tyr Ala
   190 195 200
   TTA AGC TGG TAT AAT ACG GGA TTA AAC CGT TTA CCA CGT AAT GAT GGG 855
   Leu Ser Trp Tyr Asn Thr Gly Leu Asn Arg Leu Pro Arg Asn Asp Gly
   205 210 215 220
   CTG CGA GGA TGG GCA AGA TTT AAT AGG TTT AGA AGA GAG TTA ACA ATA 903
   Leu Arg Gly Trp Ala Arg Phe Asn Arg Phe Arg Arg Glu Leu Thr Ile
                   225                 230                 235
   TCA GTA TTA GAT ATT ATT TCT TTT TTC CAA AAT TAC GAT TCT AGA TTA      951
   Ser Val Leu Asp Ile Ile Ser Phe Phe Gln Asn Tyr Asp Ser Arg Leu
               240                 245                 250
   TAT CCA ATT CCA ACA ATC TCC CAA TTA ACG CGG GAA GTA TAT ACA GAT      999
   Tyr Pro Ile Pro Thr Ile Ser Gln Leu Thr Arg Glu Val Tyr Thr Asp
           255                 260                 265
   CCG GTA ATT AAT ATA ACT GAT TAT AGA GTT ACC CCA AGT TTC GAG AGT     1047
   Pro Val Ile Asn Ile Thr Asp Tyr Arg Val Thr Pro Ser Phe Glu Ser
       270                 275                 280
   ATT GAA AAC TCA GCC ATT AGA AGC CCC CAT CTT ATG GAT TTC TTA ACT     1095
   Ile Glu Asn Ser Ala Ile Arg Ser Pro His Leu Met Asp Phe Leu Thr
   285                 290                 295                 300
   AAT ATA ATT ATT GAC ACT GAT TTA ATA AGA GGT GTT TAC TAT TGG GCA     1143
   Asn Ile Ile Ile Asp Thr Asp Leu Ile Arg Gly Val Tyr Tyr Trp Ala
                   305                 310                 315
   GGA CAT CGT ATA AAT TCT CGC TTT ACC GGG ACC GCT TTT CCA CAT ATA     1191
   Gly His Arg Ile Asn Ser Arg Phe Thr Gly Thr Ala Phe Pro His Ile
               320                 325                 330
   ATA ACA TCT CCT CAA TAT GGA ATA ACT GCA AAC GCA GAA CCA AGA CGT     1239
   Ile Thr Ser Pro Gln Tyr Gly Ile Thr Ala Asn Ala Glu Pro Arg Arg
           335                 340                 345
   ACA ATA GCG CCT GGT CCT TTT CAA GGT GTG CCT TCC CTA CTT TAT AGA     1287
   Thr Ile Ala Pro Gly Pro Phe Gln Gly Val Pro Ser Leu Leu Tyr Arg
       350                 355                 360
   ACA CTA TCA GAC CCT TTC TTC CGA AGA TCA GAC AAT ATT AGT CCA ACC     1335
   Thr Leu Ser Asp Pro Phe Phe Arg Arg Ser Asp Asn Ile Ser Pro Thr
   365                 370                 375                 380
   TTA GGG ATA AAT GTA GTA CAG GGG GTA GGG TTC TTA CAA CCA AAT AAT 1383
   Leu Gly Ile Asn Val Val Gln Gly Val Gly Phe Leu Gln Pro Asn Asn
   385 390 395
   TTT GAA TCT CTA TAT AGA AGG AGA GGG ACA GTA GAT TCT CTC GAT GAG 1431
   Phe Glu Ser Leu Tyr Arg Arg Arg Gly Thr Val Asp Ser Leu Asp Glu
   400 405 410
   TTG CCA ATT GAT GGT GAA AAT CCA TTA GTT GGA TAT AGT CAT CGA TTA 1479
   Leu Pro Ile Asp Gly Glu Asn Pro Leu Val Gly Tyr Ser His Arg Leu
   415 420 425
   AGT CAC GTT ACA TTA ACC AGG TCA TTA TTT AAT ACT AAT ATA ACT AGC 1527
   Ser His Val Thr Leu Thr Arg Ser Leu Phe Asn Thr Asn Ile Thr Ser
   430 435 440
   CTG CCA ACA TTT GTT TGG ACA CAT CAC AGT GCT ACT GAT ACA AAT ACA 1575
   Leu Pro Thr Phe Val Trp Thr His His Ser Ala Thr Asp Thr Asn Thr
   445 450 455 460
   ATT GCT CCA GAT GTC ATT ACC CAA ATA CCG TTA GTA AAG GCT TTC AAT 1623
   Ile Ala Pro Asp Val Ile Thr Gln Ile Pro Leu Val Lys Ala Phe Asn
   465 470 475
   CTT CAT TCA GGT GCC ACG GTT GCT AGA GGG CCA GGA TTT ACA GGT GGG 1671
   Leu His Ser Gly Ala Thr Val Ala Arg Gly Pro Gly Phe Thr Gly Gly
   480 485 490
   GAT ATC CTT CGA AGA ACG AAT GTT GGT AAC TTT GGA GAT ATG CGT GTA 1719
   Asp Ile Leu Arg Arg Thr Asn Val Gly Asn Phe Gly Asp Met Arg Val
   495 500 505
   AAT ATT ACT GCA CCA CTA TCA CAA AGA TAT CGA GTA AGG ATT CGT TAT 1767
   Asn Ile Thr Ala Pro Leu Ser Gln Arg Tyr Arg Val Arg Ile Arg Tyr
   510 515 520
   GCT TCT ACT ACA AAT TTA CGA TTC CAT ACA TCA ATT AAC GGA AGA GCT 1815
   Ala Ser Thr Thr Asn Leu Arg Phe His Thr Ser Ile Asn Gly Arg Ala
   525 530 535 540
   ATT AAT CAG GCG GAT TTT CCA GCT ACT ATG AAT AGT GGG GGT AAT TTA 1863
   Ile Asn Gln Ala Asp Phe Pro Ala Thr Met Asn Ser Gly Gly Asn Leu
   545 550 555
   CAG TCC GGA AGC TTC AGG ATT GCA GGT TTT ACT ACT CCA TTT ACC TTT 1911
   Gln Ser Gly Ser Phe Arg Ile Ala Gly Phe Thr Thr Pro Phe Thr Phe
   560 565 570
   TCA GAT GCA CTA AGC ACA TTC ACA ATA GGT GCT TTT GGC TTC TCT TCA 1959
   Ser Asp Ala Leu Ser Thr Phe Thr Ile Gly Ala Phe Gly Phe Ser Ser
   575 580 585
   GGT AAC GAA GTT TAT ATA GAT CGA ATT GAA TTT GTT CCG GCA GAA GTA 2007
   Gly Asn Glu Val Tyr Ile Asp Arg Ile Glu Phe Val Pro Ala Glu Val
   590 595 600
   ACC TTT GAG GCA GAA TAT GAT CTA GAA AGA GCA CAA AAG GCG GTG AAT 2055
   Thr Phe Glu Ala Glu Tyr Asp Leu Glu Arg Ala Gln Lys Ala Val Asn
   605 610 615 620
   GCG CTG TTT ACT TCT TCT AAT CAA ATC GGG TTA AAA ACA GAT GTA ACG 2103
   Ala Leu Phe Thr Ser Ser Asn Gln Ile Gly Leu Lys Thr Asp Val Thr
   625 630 635
   GAT TAT CAT ATT GAT CAA GTA TCC AAT CTA GTT GAA TGC TTA TCG GAT 2151
   Asp Tyr His Ile Asp Gln Val Ser Asn Leu Val Glu Cys Leu Ser Asp
   640 645 650
   GAA TTT TGT CTG GAT GAA AAG CGA GAA TTG TCC GAG AAA GTC AAA CAT 2199
   Glu Phe Cys Leu Asp Glu Lys Arg Glu Leu Ser Glu Lys Val Lys His
   655 660 665
   GCG AAG CGA CTC AGT GAT GAG CGG AAT TTA CTT CAA GAT CCA AAC TTT 2247
   Ala Lys Arg Leu Ser Asp Glu Arg Asn Leu Leu Gln Asp Pro Asn Phe
   670 675 680
   AGA GGG ATC AAT AGA CAA CTA GAC CGT GGC TGG AGA GGA AGT ACG GAT 2295
   Arg Gly Ile Asn Arg Gln Leu Asp Arg Gly Trp Arg Gly Ser Thr Asp
   685 690 695 700
   ATT ACC ATC CAA GGA GGA GAT GAC GTA TTC AAA GAG AAT TAC GTC ACA 2343
   Ile Thr Ile Gln Gly Gly Asp Asp Val Phe Lys Glu Asn Tyr Val Thr
   705 710 715
   CTA CCG GGT ACC TTT GAT GAG TGC TAT CCA ACG TAT TTA TAT CAA AAA 2391
   Leu Pro Gly Thr Phe Asp Glu Cys Tyr Pro Thr Tyr Leu Tyr Gln Lys
   720 725 730
   ATA GAT GAG TCA AAA TTA AAA GCC TAT ACC CGT TAT CAA TTA AGA GGG 2439
   Ile Asp Glu Ser Lys Leu Lys Ala Tyr Thr Arg Tyr Gln Leu Arg Gly
   735 740 745
   TAT ATC GAG GAT AGT CAA GAC TTA GAA ATC TAT TTA ATT CGC TAC AAT 2487
   Tyr Ile Glu Asp Ser Gln Asp Leu Glu Ile Tyr Leu Ile Arg Tyr Asn
   750 755 760
   GCA AAA CAT GAA ACA GTA AAT GTG CCA GGT ACG GGT TCT TTA TGG CCG 2535
   Ala Lys His Glu Thr Val Asn Val Pro Gly Thr Gly Ser Leu Trp Pro
   765 770 775 780
   CTT TCA GTC GAA AGT CCA ATC GGA AAG TGT GGA GAA CTA AAT CGA TGC 2583
   Leu Ser Val Glu Ser Pro Ile Gly Lys Cys Gly Glu Leu Asn Arg Cys
   785 790 795
   GCG CCA CAC CTT GAA TGG AAT CCT GAT CTA GAT TGT TCC TGC AGA GAC 2631
   Ala Pro His Leu Glu Trp Asn Pro Asp Leu Asp Cys Ser Cys Arg Asp
   800 805 810
   GGG GAA AAA TGT GCC CAT CAT TCT CAT CAT TTC TCC TTG GAC ATT GAT        2679
   Gly Glu Lys Cys Ala His His Ser His His Phe Ser Leu Asp Ile Asp
           815                     820                     825
   GTT GGA TGT ACA GAC TTA AAT GAG GAC TTA GGT GTA TGG GTG ATA TTC        2727
   Val Gly Cys Thr Asp Leu Asn Glu Asp Leu Gly Val Trp Val Ile Phe
       830                     835                     840
   AAG ATT AAG ACG CAA GAT GGC CAT GCA AGA TTA GGA AAT CTA GAA TTT        2775
   Lys Ile Lys Thr Gln Asp Gly His Ala Arg Leu Gly Asn Leu Glu Phe
   845                     850                     855                     860
   CTC GAA GAG AAA CCA TTA GTA GGA GAA GCA CTA GCT CGT GTG AAA AGA        2823
   Leu Glu Glu Lys Pro Leu Val Gly Glu Ala Leu Ala Arg Val Lys Arg
                   865                     870                     875
   GCG GAG AAA AAA TGG AGA GAC AAA CGT GAA AAA TTG GAA TTG GAA ACA        2871
   Ala Glu Lys Lys Trp Arg Asp Lys Arg Glu Lys Leu Glu Leu Glu Thr
               880                     885                     890
   AAT ATT GTT TAT AAA GAG GCA AAA GAA TCT GTA GAT GCT TTA TTT GTA        2919
   Asn Ile Val Tyr Lys Glu Ala Lys Glu Ser Val Asp Ala Leu Phe Val
           895                     900                     905
   AAC TCT CAA TAT GAT AGA TTA CAA GCG GAT ACC AAC ATC GCG ATG ATT        2967
   Asn Ser Gln Tyr Asp Arg Leu Gln Ala Asp Thr Asn Ile Ala Met Ile
       910                     915                     920
   CAT GCG GCA GAT AAA CGC GTT CAT AGC ATT CGA GAA GCT TAT CTG CCT        3015
   His Ala Ala Asp Lys Arg Val His Ser Ile Arg Glu Ala Tyr Leu Pro
   925                     930                     935                     940
   GAG CTG TCT GTG ATT CCG GGT GTC AAT GCG GCT ATT TTT GAA GAA TTA        3063
   Glu Leu Ser Val Ile Pro Gly Val Asn Ala Ala Ile Phe Glu Glu Leu
                   945                     950                     955
   GAA GGG CGT ATT TTC ACT GCA TTC TCC CTA TAT GAT GCG AGA AAT GTC 3111
   Glu Gly Arg Ile Phe Thr Ala Phe Ser Leu Tyr Asp Ala Arg Asn Val
   960 965 970
   ATT AAA AAT GGT GAT TTT AAT TAT GGC TTA TCC TGC TGG AAC GTG AAA 3159
   Ile Lys Asn Gly Asp Phe Asn Tyr Gly Leu Ser Cys Trp Asn Val Lys
   975 980 985
   GGG CAT GTA GAT GTA GAA GAA CAA AAC AAC CAC CGT TCG GTC CTT GTT 3207
   Gly His Val Asp Val Glu Glu Gln Asn Asn His Arg Ser Val Leu Val
   990 995 1000
   GTT CCG GAA TGG GAA GCA GAA GTG TCA CAA GAA GTT CGT GTC TGT CCG 3255
   Val Pro Glu Trp Glu Ala Glu Val Ser Gln Glu Val Arg Val Cys Pro
   1005 1010 1015 1020
   GGT CGT GGC TAT ATC CTT CGT GTC ACA GCT TAC AAG GAG GGA TAT GGA 3303
   Gly Arg Gly Tyr Ile Leu Arg Val Thr Ala Tyr Lys Glu Gly Tyr Gly
   1025 1030 1035
   GAA GGT TGC GTA ACC ATT CAT GAG ATC GAG AAC AAT ACA GAC GAA CTG 3351
   Glu Gly Cys Val Thr Ile His Glu Ile Glu Asn Asn Thr Asp Glu Leu
   1040 1045 1050
   AAG TTT AGC AAC TGT GTA GAA GAG GAA GTA TAT CCA AAC AAC ACG GTA 3399
   Lys Phe Ser Asn Cys Val Glu Glu Glu Val Tyr Pro Asn Asn Thr Val
   1055 1060 1065
   ACG TGT AAT GAT TAT ACT GCG ACT CAA GAA GAA TAT GAG GGT ACG TAC 3447
   Thr Cys Asn Asp Tyr Thr Ala Thr Gln Glu Glu Tyr Glu Gly Thr Tyr
   1070 1075 1080
   ACT TCT CGT AAT CGA GGA TAT GAC GGA GCC TAT GAA AGC AAT TCT TCT 3495
   Thr Ser Arg Asn Arg Gly Tyr Asp Gly Ala Tyr Glu Ser Asn Ser Ser
   1085 1090 1095 1100
   GTA CCA GCT GAT TAT GCA TCA GCC TAT GAA GAA AAA GCG TAT ACA GAT 3543
   Val Pro Ala Asp Tyr Ala Ser Ala Tyr Glu Glu Lys Ala Tyr Thr Asp
   1105 1110 1115
   GGA CGA AGA GAC AAT CCT TGT GAA TCT AAC AGA GGA TAT GGG GAT TAC 3591
   Gly Arg Arg Asp Asn Pro Cys Glu Ser Asn Arg Gly Tyr Gly Asp Tyr
   1120 1125 1130
   ACA CCA CTA CCA GCT GGC TAT GTG ACA AAA GAA TTA GAG TAC TTC CCA 3639
   Thr Pro Leu Pro Ala Gly Tyr Val Thr Lys Glu Leu Glu Tyr Phe Pro
   1135 1140 1145
   GAA ACC GAT AAG GTA TGG ATT GAG ATC GGA GAA ACG GAA GGA ACA TTT 3687
   Glu Thr Asp Lys Val Trp Ile Glu Ile Gly Glu Thr Glu Gly Thr Phe
   1150 1155 1160
   ATC GTG GAC AGC GTG GAA TTA CTC CTT ATG GAG GAA TAGTCTCATG 3733
   Ile Val Asp Ser Val Glu Leu Leu Leu Met Glu Glu
   1165 1170 1175
   CAAACACTGG TTTAAATATC GTTTTCAAAT CAATTGTCCA AGAGCAGCAT TACAAATAGA 3793
   TAAGTAATTT GTTGTAATGA AAAACGGACA TCACCTCCAT TGAAACGGAG TAGTGTCCGT 3853
   TTTACTATGT TATTTTCTAG TAATACATAT GTATAGAGCA ACTTAATCAA GCAGAGATAT 3913
   TTTCACCTAT CGATGAAAAT ATCTCTGCTT TTTCTTTTTT TTATTTGGTA TATGCTTTAC 3973
   TTGTAATCGA AAATAAAGCA CTAATAAGAG 4003
(2) INFORMATION FOR SEQ ID NO:2:
   (i) SEQUENCE CHARACTERISTICS:
      (A) LENGTH: 1176 amino acids
      (B) TYPE: amino acid
      (D) TOPOLOGY: linear
   (ii) MOLECULE TYPE: protein
   (xi) SEQUENCE DESCRIPTION: SEQ ID NO:2:
(2) INFORMATION FOR SEQ ID NO:3:
   (i) SEQUENCE CHARACTERISTICS:
      (A) LENGTH: 133 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (ix) FEATURE:
      (A) NAME/KEY: Region
      (B) LOCATION: 1..133
      (D) OTHER INFORMATION: /note= "Sub-Sequence A" from CryIE(c)
   (xi) SEQUENCE DESCRIPTION: SEQ ID NO:3:
   Ile Thr Ser Pro Gln Tyr Gly Ile Thr Ala Asn Ala Glu Pro Arg Arg
   1 5 10 15
   Thr Ile Ala Pro Gly Pro Phe Gln Gly Val Pro Ser Leu Leu Tyr Arg
   20 25 30
   Thr Leu Ser Asp Pro Phe Phe Arg Arg Ser Asp Asn Ile Ser Pro Thr
   35 40 45
   Leu Gly Ile Asn Val Val Gln Gly Val Gly Phe Leu Gln Pro Asn Asn
   50 55 60
   Phe Glu Ser Leu Tyr Arg Arg Arg Gly Thr Val Asp Ser Leu Asp Glu
   65 70 75 80
   Leu Pro Ile Asp Gly Glu Asn Pro Leu Val Gly Tyr Ser His Arg Leu
   85 90 95
   Ser His Val Thr Leu Thr Arg Ser Leu Phe Asn Thr Asn Ile Thr Ser
   100 105 110
   Leu Pro Thr Phe Val Trp Thr His His Ser Ala Thr Asp Thr Asn Thr
   115 120 125
   Ile Ala Pro Asp Val
   130
(2) INFORMATION FOR SEQ ID NO:4:
   (i) SEQUENCE CHARACTERISTICS:
      (A) LENGTH: 157 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (ix) FEATURE:
      (A) NAME/KEY: Region
      (B) LOCATION: 1..157
      (D) OTHER INFORMATION: /note= "Sub-Sequence B" from CryIE(c)
   (xi) SEQUENCE DESCRIPTION: SEQ ID NO:4:
   Ile Thr Gln Ile Pro Leu Val Lys Ala Phe Asn Leu His Ser Gly Ala
   1               5                   10                  15
   Thr Val Ala Arg Gly Pro Gly Phe Thr Gly Gly Asp Ile Leu Arg Arg
               20                  25                  30
   Thr Asn Val Gly Asn Phe Gly Asp Met Arg Val Asn Ile Thr Ala Pro
           35                  40                  45
   Leu Ser Gln Arg Tyr Arg Val Arg Ile Arg Tyr Ala Ser Thr Thr Asn
       50                  55                  60
   Leu Arg Phe His Thr Ser Ile Asn Gly Arg Ala Ile Asn Gln Ala Asp
   65                  70                  75                  80
   Phe Pro Ala Thr Met Asn Ser Gly Gly Asn Leu Gln Ser Gly Ser Phe
                   85                  90                  95
   Arg Ile Ala Gly Phe Thr Thr Pro Phe Thr Phe Ser Asp Ala Leu Ser
               100                 105                 110
   Thr Phe Thr Ile Gly Ala Phe Gly Phe Ser Ser Gly Asn Glu Val Tyr
           115                 120                 125
   Ile Asp Arg Ile Glu Phe Val Pro Ala Glu Val Thr Phe Glu Ala Glu
       130                 135                 140
   Tyr Asp Leu Glu Arg Ala Gln Lys Ala Val Asn Ala Leu
   145                 150                 155
(2) INFORMATION FOR SEQ ID NO:5:
   (i) SEQUENCE CHARACTERISTICS:
      (A) LENGTH: 13 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (ix) FEATURE:
      (A) NAME/KEY: misc_feature
      (B) LOCATION: 1..13
      (D) OTHER INFORMATION: /note= "sequence of a plant consensus translation initiator (Clontech)"
   (xi) SEQUENCE DESCRIPTION: SEQ ID NO:5:
   GTCGACCATG GTC 13
(2) INFORMATION FOR SEQ ID NO:6:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: other nucleic acid
   (iii) HYPOTHETICAL: NO
   (iv) ANTI-SENSE: NO
   (ix) FEATURE:
      (A) NAME/KEY: misc_feature
      (B) LOCATION: 1..12
      (D) OTHER INFORMATION: /note= "sequence of a plant consensus translation initiator (Joshi)"
   (xi) SEQUENCE DESCRIPTION: SEQ ID NO:6:
   TAAACAATGG CT 12
(2) INFORMATION FOR SEQ ID NO:7:
   (i) SEQUENCE CHARACTERISTICS:
      (A) LENGTH: 3531 base pairs
      (B) TYPE: nucleic acid
      (C) STRANDEDNESS: single
      (D) TOPOLOGY: linear
   (ii) MOLECULE TYPE: DNA (genomic)
   (iii) HYPOTHETICAL: NO
   (ix) FEATURE:
      (A) NAME/KEY: CDS
      (B) LOCATION: 1..3528
      (D) OTHER INFORMATION: /product= "Full-length CryIE(d) protein"
   (xi) SEQUENCE DESCRIPTION: SEQ ID NO:7:
(2) INFORMATION FOR SEQ ID NO:8:
   (i) SEQUENCE CHARACTERISTICS:
      (A) LENGTH: 1176 amino acids
      (B) TYPE: amino acid
      (D) TOPOLOGY: linear
   (ii) MOLECULE TYPE: protein
   (xi) SEQUENCE DESCRIPTION: SEQ ID NO:8:
   Met Asp Asn Asn Asn Gln Asn Gln Cys Ile Pro Tyr Asn Cys Leu Ser
   1               5                   10                  15
   Asn Pro Glu Leu Glu Ile Leu Glu Ile Glu Arg Ser Asn Asn Thr Val
               20                  25                  30
   Val Glu Asp Ile Thr Leu Gly Leu Ser Arg Leu Leu Val Ser Ala Ile
           35                  40                  45
   Pro Leu Gly Asp Phe Ile Leu Gly Leu Phe Asp Val Ile Trp Gly Ala
       50                  55                  60
   Leu Gly Arg Ser Glu Trp Asp Ile Phe Leu Glu Gln Ile Glu Leu Leu
   65                  70                  75                  80
   Ile Gly His Arg Ile Glu Glu Phe Ala Arg Asn Gln Ala Ile Ser Arg
                   85                  90                  95
   Leu Glu Gly Val Ser Asn Leu Tyr Arg Ile Tyr Thr Asn Ala Phe Lys
               100                 105                 110
   Asp Trp Glu Ala Asp Pro Thr Asn Leu Glu Leu Lys Glu Glu Met Arg
           115                 120                 125
   Thr Gln Phe Asn Asp Met Asn Ser Ala Phe Thr Thr Ala Ile Pro Leu
       130                 135                 140
   Phe Ser Val Arg Gly Tyr Glu Leu Pro Leu Leu Ser Val Tyr Val Gln
   145                 150                 155                 160
   Ala Ala Asn Leu His Leu Ser Val Leu Arg Asp Val Ser Val Phe Gly
                   165                 170                 175
   Gln Arg Trp Gly Phe Asp Val Ala Thr Ala Asn Arg Arg Tyr Asp Asp
               180                 185                 190
   Leu Thr Thr Asn Ile Gly Asp Tyr Thr Asp Tyr Ala Leu Ser Trp Tyr
           195                 200                 205
   Asn Thr Gly Leu Thr Arg Leu Pro Arg Asn Asp Gly Leu Arg Gly Trp
       210                 215                 220
   Ala Arg Phe Ile Arg Phe Arg Arg Glu Leu Thr Ile Ser Val Leu Asp
   225                 230                 235                 240
   Ile Ile Ser Phe Phe Gln Asn Tyr Asp Ser Arg Leu Tyr Pro Ile Pro
                   245                 250                 255
   Thr Ile Ser Gln Leu Thr Arg Glu Val Tyr Thr Asp Pro Val Ile Asn
               260                 265                 270
   Ile Thr Asp Tyr Arg Val Thr Pro Ser Phe Glu Ser Ile Glu Asn Ser
           275                 280                 285
   Ala Ile Arg Ser Pro His Leu Met Asp Phe Leu Thr Asn Ile Ile Ile
       290                 295                 300
   Asp Thr Asp Leu Ile Arg Gly Val Tyr Tyr Trp Ala Gly His Arg Ile
   305                 310                 315                 320
   Asn Ser Arg Phe Thr Gly Thr Ala Phe Pro His Ile Ile Thr Ser Pro
                   325                 330                 335
   Gln Tyr Gly Ile Thr Ala Asn Ala Glu Pro Arg Arg Thr Ile Ala Pro
               340                 345                 350
   Gly Pro Phe Gln Gly Val Pro Ser Leu Leu Tyr Arg Thr Leu Ser Asp
   355 360 365
   Pro Phe Phe Arg Arg Ser Asp Asn Ile Ser Pro Thr Leu Gly Ile Asn
   370 375 380
   Val Val Gln Gly Val Gly Phe Leu His Pro Asn Asn Phe Glu Ser Leu
   385 390 395 400
   Tyr Arg Arg Arg Gly Thr Val Asp Ser Leu Asp Glu Leu Pro Ile Asp
   405 410 415
   Gly Glu Asn Pro Leu Val Gly Tyr Ser His Arg Leu Ser His Val Thr
   420 425 430
   Leu Thr Arg Ala Leu Phe Asn Ser Asn Ile Thr Ser Leu Pro Thr Phe
   435 440 445
   Val Trp Thr His Arg Ser Ala Thr Asp Thr Lys Thr Ile Ala Pro Asp
   450 455 460
   Val Ile Thr Gln Ile Pro Leu Val Lys Ala Phe Asn Leu His Ser Gly
   465 470 475 480
   Ala Pro Val Ala Arg Gly Pro Gly Phe Thr Gly Gly Asp Ile Leu Arg
   485 490 495
   Arg Thr Asn Val Gly Asn Phe Gly Asp Ile Arg Val Asn Ile Thr Ala
   500 505 510
   Pro Leu Ser Gln Arg Tyr Arg Val Arg Ile Arg Tyr Ala Ser Thr Thr
   515 520 525
   Asn Leu Arg Phe His Thr Ser Ile Asn Gly Arg Ala Ile Asn Gln Ala
   530 535 540
   Asp Phe Pro Ala Thr Met Asn Ser Gly Gly Asn Leu Gln Ser Gly Ser
   545 550 555 560
   Phe Arg Ile Ala Gly Phe Thr Thr Pro Phe Thr Phe Ser Asp Ala Leu
   565 570 575
   Ser Thr Phe Thr Ile Gly Ala Phe Gly Phe Ser Ser Gly Asn Glu Val
   580 585 590
   Tyr Ile Asp Arg Ile Glu Phe Val Pro Ala Glu Val Thr Phe Glu Ala
   595 600 605
   Glu Tyr Asp Leu Glu Arg Ala Gln Lys Ala Val Asn Ala Leu Phe Thr
   610 615 620
   Ser Ser Asn Gln Ile Gly Leu Lys Thr Asp Val Thr Asp Tyr His Ile
   625 630 635 640
   Asp Gln Val Ser Asn Leu Val Glu Cys Leu Ser Asp Glu Phe Cys Leu
   645 650 655
   Asp Glu Lys Arg Glu Leu Ser Glu Lys Val Lys His Ala Lys Arg Leu
   660 665 670
   Ser Asp Glu Arg Asn Leu Leu Gln Asp Pro Asn Phe Arg Gly Ile Asn
   675 680 685
   Arg Gln Leu Asp Arg Gly Trp Arg Gly Ser Thr Asp Ile Thr Ile Gln
   690 695 700
   Gly Gly Asp Asp Val Phe Lys Glu Asn Tyr Val Thr Leu Pro Gly Thr
   705 710 715 720
   Phe Asp Glu Cys Tyr Pro Thr Phe Leu Tyr Gln Lys Ile Asp Glu Ser
   725 730 735
   Lys Leu Lys Ala Tyr Thr Arg Tyr Gln Leu Arg Gly Tyr Ile Glu Asp
   740 745 750
   Ser Gln Asp Leu Glu Ile Tyr Leu Ile Arg Tyr Asn Ala Lys His Glu
   755 760 765
   Thr Val Asn Val Pro Gly Thr Gly Ser Leu Trp Pro Leu Ser Val Glu
   770 775 780
   Ser Pro Ile Gly Lys Cys Gly Glu Leu Asn Arg Cys Ala Pro His Leu
   785 790 795 800
   Glu Trp Asn Pro Asp Leu Asp Cys Ser Cys Arg Asp Gly Glu Lys Cys
   805 810 815
   Ala His His Ser His His Phe Ser Leu Asp Ile Asp Val Gly Cys Thr
   820 825 830
   Asp Leu Asn Glu Asp Leu Gly Val Trp Val Ile Phe Lys Ile Lys Thr
   835 840 845
   Gln Asp Gly His Ala Arg Leu Gly Asn Leu Glu Phe Leu Glu Glu Lys
   850 855 860
   Pro Leu Val Gly Glu Ala Leu Ala Arg Val Lys Arg Ala Glu Lys Lys
   865 870 875 880
   Trp Arg Asp Lys Arg Glu Lys Leu Glu Leu Glu Thr Asn Ile Val Tyr
   885 890 895
   Lys Glu Ala Lys Glu Ser Val Asp Ala Leu Phe Val Asn Ser Gln Tyr
   900 905 910
   Asp Arg Leu Gln Ala Asp Thr Asn Ile Ala Met Ile His Ala Ala Asp
   915 920 925
   Lys Arg Val His Ser Ile Arg Glu Ala Tyr Leu Pro Glu Leu Ser Val
   930 935 940
   Ile Pro Gly Val Asn Ala Ala Ile Phe Glu Glu Leu Glu Gly Arg Ile
   945 950 955 960
   Phe Thr Ala Phe Ser Leu Tyr Asp Ala Arg Asn Val Ile Lys Asn Gly
   965 970 975
   Asp Phe Asn Tyr Gly Leu Ser Cys Trp Asn Val Lys Gly His Val Asp
   980 985 990
   Val Glu Glu Gln Asn Asn His Arg Ser Val Leu Val Val Pro Glu Trp
   995 1000 1005
   Glu Ala Glu Val Ser Gln Glu Val Arg Val Cys Pro Gly Arg Gly Tyr
   1010 1015 1020
   Ile Leu Arg Val Thr Ala Tyr Lys Glu Gly Tyr Gly Glu Gly Cys Val
   1025 1030 1035 1040
   Thr Ile His Glu Ile Glu Asn Asn Thr Asp Glu Leu Lys Phe Ser Asn
   1045 1050 1055
   Cys Val Glu Glu Glu Val Tyr Pro Asn Asn Thr Val Thr Cys Asn Asp
   1060 1065 1070
   Tyr Thr Ala Thr Gln Glu Glu Tyr Glu Gly Thr Tyr Thr Ser Arg Asn
   1075 1080 1085
   Arg Gly Tyr Asp Gly Ala Tyr Glu Ser Asn Ser Ser Val Pro Ala Asp
   1090 1095 1100
   Tyr Ala Ser Ala Tyr Glu Glu Lys Ala Tyr Thr Asp Gly Arg Arg Asp
   1105 1110 1115 1120
   Asn Pro Cys Glu Ser Asn Arg Gly Tyr Gly Asp Tyr Thr Pro Leu Pro
   1125 1130 1135
   Ala Gly Tyr Val Thr Lys Glu Leu Glu Tyr Phe Pro Glu Thr Asp Lys
   1140 1145 1150
   Val Trp Ile Glu Ile Gly Glu Thr Glu Gly Thr Phe Ile Val Asp Ser
           1155                1160                1165
   Val Glu Leu Leu Leu Met Glu Glu
       1170                1175
(2) INFORMATION FOR SEQ ID NO:9:
   (i) SEQUENCE CHARACTERISTICS:
      (A) LENGTH: 133 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (ix) FEATURE:
      (A) NAME/KEY: Region
      (B) LOCATION: 1..133
      (D) OTHER INFORMATION: /note="Sub-Sequence A' from CryIE(d)"
   (xi) SEQUENCE DESCRIPTION: SEQ ID NO:9:
   Ile Thr Ser Pro Gln Tyr Gly Ile Thr Ala Asn Ala Glu Pro Arg Arg
   1               5                   10                  15
   Thr Ile Ala Pro Gly Pro Phe Gln Gly Val Pro Ser Leu Leu Tyr Arg
               20                  25                  30
   Thr Leu Ser Asp Pro Phe Phe Arg Arg Ser Asp Asn Ile Ser Pro Thr
   35 40 45
   Leu Gly Ile Asn Val Val Gln Gly Val Gly Phe Leu His Pro Asn Asn
   50 55 60
   Phe Glu Ser Leu Tyr Arg Arg Arg Gly Thr Val Asp Ser Leu Asp Glu
   65 70 75 80
   Leu Pro Ile Asp Gly Glu Asn Pro Leu Val Gly Tyr Ser His Arg Leu
   85 90 95
   Ser His Val Thr Leu Thr Arg Ala Leu Phe Asn Ser Asn Ile Thr Ser
   100 105 110
   Leu Pro Thr Phe Val Trp Thr His Arg Ser Ala Thr Asp Thr Lys Thr
   115 120 125
   Ile Ala Pro Asp Val
   130
(2) INFORMATION FOR SEQ ID NO:10:
   (i) SEQUENCE CHARACTERISTICS:
      (A) LENGTH: 157 amino acids
      (B) TYPE: amino acid
      (C) STRANDEDNESS: single
      (D) TOPOLOGY: linear
   (ii) MOLECULE TYPE: protein
   (iii) HYPOTHETICAL: NO
   (iv) ANTI-SENSE: NO
   (ix) FEATURE:
      (A) NAME/KEY: Region
      (B) LOCATION: 1..157
      (D) OTHER INFORMATION: /note= "Sub-Sequence B' from CryIE(d)"
   (xi) SEQUENCE DESCRIPTION: SEQ ID NO:10:
   Ile Thr Gln Ile Pro Leu Val Lys Ala Phe Asn Leu His Ser Gly Ala
   1               5                   10                  15
   Pro Val Ala Arg Gly Pro Gly Phe Thr Gly Gly Asp Ile Leu Arg Arg
               20                  25                  30
   Thr Asn Val Gly Asn Phe Gly Asp Ile Arg Val Asn Ile Thr Ala Pro
           35                  40                  45
   Leu Ser Gln Arg Tyr Arg Val Arg Ile Arg Tyr Ala Ser Thr Thr Asn
       50                  55                  60
   Leu Arg Phe His Thr Ser Ile Asn Gly Arg Ala Ile Asn Gln Ala Asp
   65                  70                  75                  80
   Phe Pro Ala Thr Met Asn Ser Gly Gly Asn Leu Gln Ser Gly Ser Phe
                   85                  90                  95
   Arg Ile Ala Gly Phe Thr Thr Pro Phe Thr Phe Ser Asp Ala Leu Ser
               100                 105                 110
   Thr Phe Thr Ile Gly Ala Phe Gly Phe Ser Ser Gly Asn Glu Val Tyr
           115                 120                 125
   Ile Asp Arg Ile Glu Phe Val Pro Ala Glu Val Thr Phe Glu Ala Glu
       130                 135                 140
   Tyr Asp Leu Glu Arg Ala Gln Lys Ala Val Asn Ala Leu
   145                 150                 155
(2) INFORMATION FOR SEQ ID NO:11:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: primer SON0003 used to construct pSOG10
   (iii) HYPOTHETICAL: NO
   (iv) ANTI-SENSE: NO
   (xi) SEQUENCE DESCRIPTION: SEQ ID NO:11:
   CTCGGATCCAGCAGATTCGAAGAAGGTACAG                                   31
(2) INFORMATION FOR SEQ ID NO:12:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: primer SON0004 used to construct pSOG10
   (iii) HYPOTHETICAL: NO
   (iv) ANTI-SENSE: NO
   (xi) SEQUENCE DESCRIPTION: SEQ ID NO:12:
   ACGGGATCCAACTTCCTAGCTGAAAAATGGG 31
(2) INFORMATION FOR SEQ ID NO:13:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: primer SON0031 used to construct pSOG19
   (iii) HYPOTHETICAL: NO
   (iv) ANTI-SENSE: NO
   (xi) SEQUENCE DESCRIPTION: SEQ ID NO:13:
   CATGAGGGACTGACCACCCGGGGATC 26
(2) INFORMATION FOR SEQ ID NO:14:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: primer SON0010 used to construct pSOG19
   (iii) HYPOTHETICAL: NO
   (iv) ANTI-SENSE: NO
   (xi) SEQUENCE DESCRIPTION: SEQ ID NO:14:
   AGCGGATAACAATTTCACACAGGA 24
(2) INFORMATION FOR SEQ ID NO:15:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: primer SON0016 used to construct pSOG19
   (iii) HYPOTHETICAL: NO
   (iv) ANTI-SENSE: NO
   (xi) SEQUENCE DESCRIPTION: SEQ ID NO:15:
   GCTACCATGGCCACATAGAACACC 24
(2) INFORMATION FOR SEQ ID N0:16:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: primer SON0017 used to construct pSOG19
   (iii) HYPOTHETICAL: NO
   (iv) ANTI-SENSE: NO
   (xi) SEQUENCE DESCRIPTION: SEQ ID NO:16:
(2) INFORMATION FOR SEQ ID NO:17:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: primer SON0039 used to construct pSOG30
   (iii) HYPOTHETICAL: NO
   (iv) ANTI-SENSE: NO
   (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: primer SON0041 used to construct pSOG30
   (iii) HYPOTHETICAL: NO
   (iv) ANTI-SENSE: NO
   (xi) SEQUENCE DESCRIPTION: SEQ ID NO:18:
   ATCGCAAGACCGGCAACAGGATTC 24

## Description

The present invention relates to novel toxin genes isolated from *Bacillus thuringiensis* var. *kurstaki,* to the proteins encoded by the genes, recombinant strains comprising at least one of the novel toxin genes and entomocidal compositions containing at least one of the recombinant strains, as well as transgenic plants comprising at least one of the novel toxin genes or their derivatives.

*Bacillus thuringiensis* belongs to the large group of gram-positive, aerobic, endospore-forming bacteria. Unlike other very closely related species of *Bacillus* such as *B*. *cereus* or *B. anthracis,* the majority of the hitherto known *Bacillus thuringiensis* species produce in the course of their sporulation a parasporal inclusion body which, on account of its crystalline structure, is generally referred to also as a crystalline body. This crystalline body is composed of insecticidally active crystalline protoxin proteins, the so-called δ-endotoxins.

These protein crystals are responsible for the toxicity to insects of *Bacillus thuringiensis.* The δ-endotoxin does not exhibit its insecticidal activity until after oral intake of the crystalline body, when the latter is dissolved in the intestinal juice of the target insects. In most cases the actual toxic component is released from the protoxin as a result of proteolytic cleavage caused by the action of proteases from the digestive tract of the insects.

The δ-endotoxins of the various *Bacillus thuringiensis* strains are characterized by high specificity with respect to certain target insects, especially with respect to various Lepidoptera, Coleoptera and Diptera larvae, and by a high degree of activity against these larvae. A further advantage in using δ-endotoxins of *Bacillus thuringiensis* resides in the fact that the toxins are harmless to humans, other mammals, birds and fish.

With the introduction of genetic engineering and the new possibilities resulting from it, the field of *Bacillus thuringiensis* toxins has received a fresh impetus. For example, it is known that many naturally-occurring strains possess more than one insect toxin protein, which may account for a wide spectrum of insecticidal activity of those strains. However, with the ability to genetically transform *Bacillus* it is possible to create recombinant strains which may contain a chosen array of insect toxin genes obtained by isolation and cloning from naturally-occurring sources. Such recombinant strains can be made to provide whatever spectrum of insecticidal activity might be desired for a particular application, based upon a knowledge of the insecticidal activity of individual toxin proteins. Furthermore, it is also possible to create recombinant toxin proteins which have a chosen combination of functions designed to enhance the degree of insecticidal activity against a particular insect or insect class, or to expand the spectrum of insects against which the toxin protein is active.

The various insecticidal crystal proteins from *Bacillus thuringiensis* have been classified based upon their spectrum of activity and sequence similarity. The classification put forth by Höfte and Whiteley, Microbiol. Rev. 53: 242-255 (1989) placed the then known insecticidal crystal proteins into four major classes. Generally, the major classes are defined by the spectrum of activity, with the Cryl proteins active against Lepidoptera, Cryll proteins active against both Lepidoptera and Diptera, Crylll proteins active against Coleoptera, and CrylV proteins active against Diptera.

Within each major class, the δ-endotoxins are grouped according to sequence similarity. The Cryl proteins are typically produced as 130-140 kDa protoxin proteins which are proteolytically cleaved to produce active toxin proteins about 60-70 kDa. The active portion of the δ-endotoxin resides in the NH₂-terminal portion of the full-length molecule. Höfte and Whiteley, *supra,* classified the then known Cryl proteins into six groups, IA(a), IA(b), IA(c), IB, IC, and ID. Since then, proteins dassified as CrylE, CrylF, CrylG, CrylH and CrylX have also been characterized.

Moar WJ *et al.* (Applied and environmental microbiology, volume 60(3), pages 896 to 902) describes the insecticidal activity of the CryIIA protein from the NRD-12 isolate of *Bacillus thuringiensis* subspecies *kurstaki.*

Van Frankenhuyzen *et al.* (Journal of Invertebrate Pathology, volume 62, pages 295-301) compares the insecticidal activity of Cry1B, Cry1C, Cry1D and Cry1E delta-endotoxin proteins from *Bacillus thutingiensis* against that of Cry1A toxins for a range of insect species.

Honée *et al.* (Molecular Microbiology, volume 5(11), pages 2799-2806) relates to binding studies of hybrid crystal proteins based on Cry1A(b) and Cry1C.

The spectrum of insecticidal activity of an individual δ-endotoxin from *Bacillus thuringiensis* tends to be quite narrow, with a given δ-endotoxin being active against only a few insects. Specificity is the result of the efficiency of the various steps involved in producing an active toxin protein and its subsequent ability to interact with the epithelial cells in the insect digestive tract.

To be insecticidal, a δ-endotoxin must first be ingested by the insect, solubilized and in most cases proteolytically cleaved to form an active toxin. Once activated, the δ-endotoxins bind to specific proteins present on the surface of the insect's gut epithelial cells through the agency of a specific domain on the protein toxin. In all cases examined, binding of the protein toxin to the insect gut occurs whenever there is toxicity. After binding, a different domain of the δ-endotoxin is thought to insert itself into the cell membrane creating a pore that results in the osmotic rupture of the insect's gut epithelial cell. The protein toxin's specificity to particular insects is thought to be determined, in large part, by the properties of the binding domain of an activated δ-endotoxin.

The size of the region of a δ-endotoxin required for binding to the insect gut cell binding protein is unclear. Schnepf *et al*. (J. Biol. Chem. 265: 20923-20930, 1990) have shown that the region between amino acids 332 and 722 contributes substantially to the specificity of CrylA(c) toward *Heliothis virescens* and that there are sub-sequences within this region which additively contribute to this specificity. This region of amino acids 332 to 722 spans both the specificity determining "Domain II" and the structural orientation determining "Domain III" as defined by Li *et al.* (Nature 353: 815-821, 1991). The importance of sub-sequences within this broad region is further emphasized by the findings of Ge *et al*. (Proc. Natl. Acad. Sci. USA, 86: 4037-4041, 1989), who identified a region of CrylA(a) from amino acids 332 to 450 that determined the specificity of the protein's toxicity toward the silkworm (*Bombyx morr*, Lepidoptera).

It is an objective of the present invention to provide novel genes encoding a toxin protein and entomocidal compositions comprising the same.

In particular, the present invention is drawn to novel toxin genes of the CryIE- type obtainable from *Bacillus thuringiensis* var *kurstaki,* which toxin genes encode a CryIE-type toxin protein which is active against *Heliothis* species but does not exhibit an activity against *Spodoptera exigua*.

Each of the novel toxin genes encode a protein of approximately 130 kDa in size and is active against Lepidopteran insects. The novel toxin genes have been given the designation CryIE(c) [SEQ ID NO:1] and CryIE(d) [SEQ ID NO: 7]

Hence, the present invention particularly relates to an isolated DNA molecule encoding the toxin protein CryIE(c) and CryIE(d), respectively and having the nucleotide sequence set forth in SEQ ID NO:1 and SEQ ID NO 7, respectively. Further comprised is an isolated DNA molecule that has been truncated to encode the toxic core fragment of the toxin protein CrylE(c) and CrylE(d), respectively.

Also included in the invention are the proteins produced by CrylE(c) [SEQ ID NO:2] and CrylE(d) [SEQ ID NO 8], respectively and/or the toxic core fragment of the said toxin proteins.

The invention further encompasses recombinant genes and proteins possessing one or more of the sub-sequences of the novel genes which confer activity against insects of the genus *Heliothis.*

It is thus a further object of the invention to provide an isolated DNA molecule encoding a toxin protein wherein said toxin protein comprises a sequence selected from the group consisting of Sub-Sequence A (SEQ ID NO:3) and Sub-Sequence B (SEQ ID NO:4) obtainable from CrylE(c) or Sub-Sequence A' (SEQ ID NO:9) and Sub-Sequence B' (SEQ ID NO:10) obtainable from CrylE(d), respectively and is active against *Heliothis* species but does not exhibit an activity against *Spodoptera exigua*. Also included are isolated DNA molecules wherein said toxin protein comprises both Sub-Sequence A (SEQ ID NO:3) and Sub-Sequence B (SEQ ID NO:4) obtainable from CrylE(c) or Sub-Sequence A' (SEQ ID NO:9) and Sub-Sequence B' (SEQ ID NO:10) obtainable from CrylE(d), respectively, or combinations thereof. The proteins being encoded by the said DNA molecules are also included in the scope of the invention.

A specific embodiment of the invention relates to an isolated DNA molecule encoding the toxin protein CrylE(c) and CrylE(d), respectively and/or an isolated DNA molecule that has been truncated to encode the toxic core fragment of the said toxin proteins, which DNA molecule has been optimized for expression in plants, but especially in maize plants.

The invention further encompasses recombinant genes and proteins possessing one or more of the sub-sequences of the CryIE-type genes according to the invention,but especially to sub-sequences selected from the group consisting of Sub-Sequence A (SEQ ID NO:3) and Sub-Sequence B (SEQ ID NO:4) obtainable from CryIE(c) or Sub-Sequence A' (SEQ ID NO:9) and Sub-Sequence B' (SEQ ID NO:10) obtainable from CryIE(d), respectively, which confer activity against insects of the genus *Heliothis*.

In particular, the invention relates to a recombinant DNA molecule comprising a DNA molecule encoding a toxin protein of the CrylE type, which is active against *Heliothis* species but does not exhibit an activity against *Spodoptera exigua,* but especially a CrylE(c) and CryIE(d) protein as given in SEQ ID NO: 2 and SEQ ID NO: 8, respectively. In a preferred embodiment, the invention relates to a recombinant DNA molecule comprising a chimeric construct, wherein the DNA molecule encoding a toxin protein of the CrylE type which is active against *Heliothis* species but does not exhibit an activity against *Spodoptera exigua* is under the control of expression sequences that are operable in microorganisms and/or plants.

An additional aspect of the invention are recombinant, biologically pure microbial strains transformed with at least one of the novel genes which can be used in entomocidal formulations for the control of Lepidopteran insects. In a specific embodiment the invention relates to microorgansims selected from the group consisting of members of the genus *Bacillus, Pseudomonas, Caulobacter, Agmellenum, Rhizobium,* and *Clavibacter;* to baculoviruses such as, for example the nuclear polyhedrosis virus *Autographica californica* and to yeast, e.g., *Saccharomyces, Cryptococcus, Kluyveromyces, Sporobolomyces, Rhodotorula,* and *Aureobasidium..*

The invention thus further relates to entomocidal compositions comprising at least one of the novel CryIE-type toxin proteins, but especially an isolated protein molecule having the sequence set forth in SEQ ID NO: 2 and/or an isolated protein molecule having the sequence set forth in SEQ ID NO: 8 and/or an isolated protein molecule comprising a sequence selected from the group consisting of Sub-Sequence A (SEQ ID NO:3), Sub-Sequence B (SEQ ID NO:4), Sub-Sequence A' (SEQ ID NO:9), Sub-Sequence B' (SEQ ID NO:10) and a combination of Sub-Sequence A, B, A' and B' wherein said toxin protein is active against *Heliothis* species, in an insecticidally-effective amount together with a suitable carrier.

In a preferred embodiment the invention relates to an entomocidal composition comprising a recombinant microorganism, but especially a recombinant *Bacillus thuringiensis* strain, containing at least one of the novel toxin genes in recombinant form or a derivative or mutant thereof.

Yet another aspect of the invention are plants transformed with at least one of the novel toxin genes or active fragments thereof, particularly where the transforming sequences have been optimized for expression in plants, but especially in maize plants.

In particular, the invention relates to a transformed plant comprising a DNA molecule encoding the toxin protein CrylE(c) and CrylE(d), respectively, or a combination thereof and having the nucleotide sequence set forth in SEQ ID NO:1 and SEQ ID NO 7, respectively and/or an isolated DNA molecule that has been truncated to encode the toxic core fragment of the said toxin proteins.

The invention further relates to a transformed plant comprising a DNA molecule encoding a toxin protein wherein said toxin protein comprises a sequence selected from the group consisting of Sub-Sequence A (SEQ ID NO:3), Sub-Sequence B (SEQ ID NO:4), Sub-Sequence A' (SEQ ID NO:9), Sub-Sequence B' (SEQ ID NO:10) and a combination of Sub-Sequence A, B, A' and B' wherein said toxin protein is active against *Heliothis* species.

Preferred within the invention are transformed plants selected from the group consisting of maize, wheat, barley, rice, tobacco, cotton, and soybean.

The invention further relates to methods of producing an isolated DNA molecule according to the invention or of a toxin protein being encoded by the said DNA molecule. Methods of producing recombinant microorganisms and plants comprising the said DNA molecule are also embodied by the present invention as well as methods for producing entomocidal compositions.

In particular, the invention relates to a method of obtaining an isolated DNA molecule encoding a toxin protein of the CrylE-type, but especially of the CryIE(c) and CrylE(d) type, respectively which is active against *Heliothis* species but does not exhibit an activity against *Spodoptera exigua*, which method comprises
(a) isolating total DNA from *Bacillus thuringiensis* var *kurstaki;*
(b) establishing a genomic DNA library in a suitable host organism:
(c) probing the said library with a suitable probe molecule;
(d) isolating a done that hybridizes with the probe molecule; and
(e) assaying the said done for insecticidal activity.

The invention further relates to a method of producing an isolated protein molecule of the CrylE type, but especially of the CryIE(c) and CryIE(d) type, respectively which is active against *Heliothis* species but does not exhibit an activity against *Spodoptera exigua*, which method comprises
(a) transforming a suitable host organism with a recombinant DNA molecule comprising a chimeric construct, wherein the DNA molecule encoding a toxin protein of the CrylE type which is active against *Heliothis* species but does not exhibit an activity against *Spodoptera exigua* is under the control of expression sequences that are operable in the said host organism;
(b) growing the so transformed host organism in a suitable medium; and
(c) isolating the recombinant protein product produced by the transfomred host organism upon expression of the toxin gene.

The invention further encompases a method of producing a transgenic plant comprising transforming the said plant with a recombinant DNA molecule comprising a chimeric construct, wherein the DNA molecule encoding a toxin protein of the CrylE-type, but especially of the CrylE(c) and CrylE(d) type, respectively which is active against *Heliothis* species but does not exhibit an activity against *Spodoptera exigua* is under the control of expression sequences that are operable in the said plant.

It is a further object of the invention to provide a method of producing a recombinant microorganism comprising transforming the said microorganism with a recombinant DNA molecule comprising a chimeric construct, wherein the DNA molecule encoding a toxin protein of the CryIE-type which is active against *Heliothis* species but does not exhibit an activity against *Spodoptera exigua* is under the control of expression sequences that are operable in the said microorganism.

The invention also relates to a method of producing an entomocidal composition comprising mixing a recombinant microorganism or an isolated protein molecule according to the invention in an insecticidally-effective amount with a suitable carrier.

Its is a further object of the invention to provide methods for protecting plants against damage caused by insect pest comprising applying to the plant or the growing area at least one of the novel toxin proteins according to the invention either directly or in form of an entomocidal composition comprising at least one of the said proteins or a a recombinant microorganism, but especially a recombinant *Bacillus thuringiensis* strain, containing at least one of the novel toxin genes in recombinant form or a derivative or mutant thereof. In a further embodiment, a transgenic plant according to the invention may be used in a method of protecting plants against damage caused by an insect pest comprising planting a plant according to the invention within an area where the said insect pest may occur.

The isolation and purification of the novel toxin genes of the present invention is described at length in Example 1. According to the nomenclature scheme of Höfte and Whiteley, Microbiol. Rev. 53: 242-255 (1989), the proteins encoded by the novel toxin genes of the present invention would be classified as a CryIE-type, and is designated herein as CryIE(c). The coding region of the novel toxin gene (SEQ ID NO:1) extends from nucleotide base position 196 to position 3723. The protein produced by the novel toxin gene CrylE(c) has the amino acid sequence disclosed as SEQ ID NO: 2, and is further characterized in Example 5.

The coding region of the novel toxin gene CryIE(d) (SEQ ID NO:7) extends from nucleotide base position 1 to position 3528. The protein produced by the novel toxin gene CrylE(d) has the amino acid sequence disclosed as SEQ ID NO:8, and is also further characterized in Example 5. The size of both the CrylE(c) protein (SEQ ID NO:2) and the CrylE(d) protein (SEQ ID NO:8), as deduced from their DNA sequence, is 130.7 kDa. This is similar in size to other known CrylE-type proteins. Despite the apparent size similarity, comparison of the amino acid sequence of the proteins encoded by the novel toxin genes with that of the previously reported CryIE-type protein shows significant differences between the two. The CryIE(c) and CryIE(d) proteins have only 81 % similarity to CryIE(a) protein and only 83 % similarity to the CryIE(b) protein.

The novel toxin genes CryIE(c) and CryIE(d) are 99 % identical to each other at the protein level, differing in amino acid residue only at the positions indicated in Table 1.

**Table 1: Differences in amino acid composition between the CrylE(c) protein (SEQ ID NO:2) and the CrylE(d) protein (SEQ ID NO:8).**

| ***Residue Position*** | ***Amino Acid in CrylE(c)*** | ***Amino Acid in CrylE(d)*** |
|---|---|---|
| 84 | Glutamine | Histidine |
| 187 | Valine | Alanine |
| 214 | Asparagine | Threonine |
| 229 | Asparagine | Isoleucine |
| 394 | Glutamine | Histidine |
| 437 | Serine | Alanine |
| 441 | Threonine | Serine |
| 453 | Histidine | Arginine |
| 459 | Asparagine | Lysine |
| 483 | Threonine | Proline |
| 506 | Methionine | Isoleucine |
| 729 | Tyrosine | Phenylalanine |

A comparison of the nucleotide base sequence of the CrylE(c) and the CrylE(d) genes of the present invention with the corresponding coding regions of other CrylE-type genes known in the art indicate significant differences between them. The CryIE(c) and CryIE(d) genes of the present invention have only 87 % sequence identity to the known endotoxin gene designated CryIE(a) and only 85 % sequence identity to the known endotoxin gene designated CryIE(b).

While the CrylE(c) and CryIE(d) proteins of the present invention show limited sequence similarity with other known CryIE-type proteins, they exhibit insecticidal activities distinct from that of the known CryIE-type proteins. As shown below in Example 4 and Table 3, the proteins encoded by the novel toxin genes of the present invention are active against the Lepidopteran insect *Heliothis virescens.* Previously reported CrylE-type proteins are not known to be active against this insect pest. Furthermore, the previously reported CrylE-type proteins are known to be active against the Lepidopteran insect *Spodoptera exigua* whereas the CrylE(c) and CrylE(d) proteins of the present invention do not possess this activity.

CrylE(d) further possesses activity against *Spodoptera frugiperda*. Finally, both CrylE(c) and CrylE(d) proteins are active against the insect *Plutella xylostella* whereas the previously known CrylE-type proteins are not toxic to that particular insect.

The unique insecticidal activity of the CrylE(c) and CrylE(d) proteins of the present invention against *Heliothis virescens* is the result of two sub-sequences located in the region spanning amino acid residues 332 to 622 of the protein. This region corresponds to regions identified in other δ-endotoxin proteins as responsible for insect specificity ("Domain II") and structural orientation ("Domain III"). *See* Li *et al*. (Nature 353: 815-821, 1991). Sub-sequences within this region correspond to the domains described by Li *et al*., *supra.*

The first sub-sequences identified, Sub-Sequence A [SEQ ID NO:3); from CrylE(c)]. and Sub-Sequence A' [SEO ID NO:9; from CrylE(d)], spans the region from amino acids 332 to 465. It is characterized as having low sequence similarity to other Cryl-type toxin proteins yet is suggested to additively determine activity against *Heliothis virescens* based on the work of Schnepf *et al*., (J. Biol. Chem. 265: 20923-20930, 1990). Sub-Sequence A also corresponds to the specificity determining "Domain II" as described by Li *et al*., (Nature 353: 815-821, 1991).

The second sub-sequence identified, Sub-Sequence B (SEQ ID NO:4), from CrylE(c)]. and Sub-Sequence B' [SEQ ID NO:10; from CrylE(d)], spans the region from amino acids 466 to 622. This region is characterized by its high sequence similarity with the corresponding region found in another protein from *Bacillus thuringiensis,* CrylA(a), which is as active on *Heliothis virescens* as the novel toxin protein CryIE(c) or CryIE(d). Sub-Sequence B corresponds to the strutural orientation "Domain III" as described by U *et al*., (Nature 353: 815-821, 1991). The similarity between Sub-Sequence B in CrylE(c) and the corresponding region in CrylA(a) for the two proteins is shown in Table 2, below.

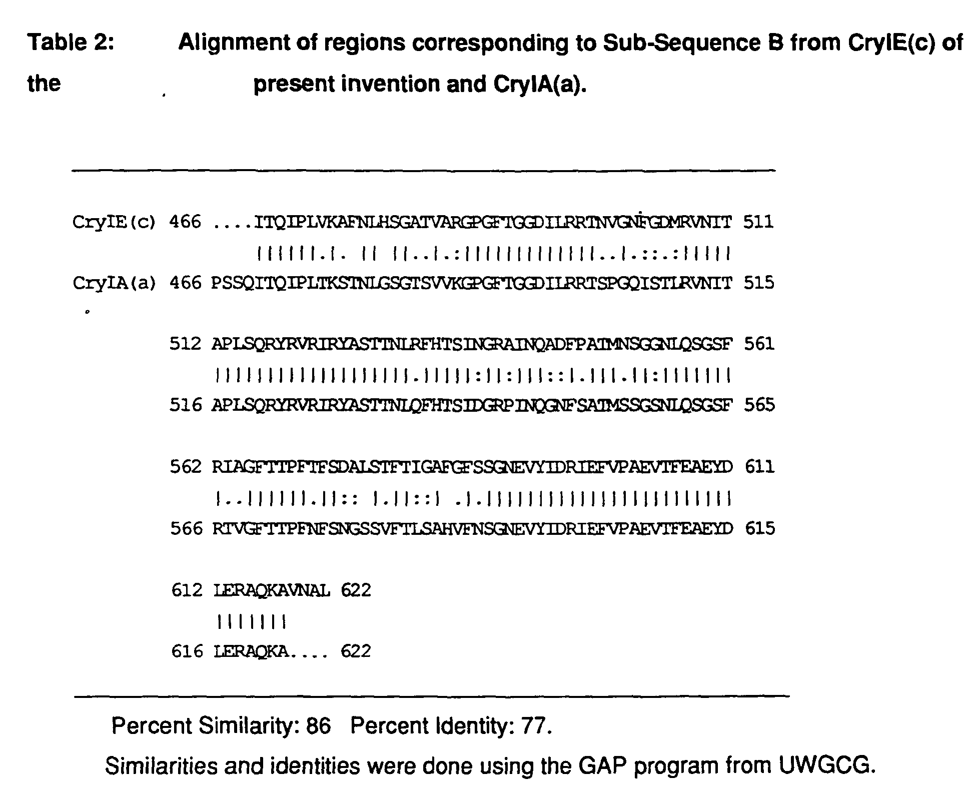

The high percent similarity (86 %) between the Sub-Sequence B region of the novel toxin protein CryIE(c) of the present invention and the corresponding region of the CrylA(a) protein indicates the conserved nature of the region which confers toxicity to *Heliothis virescens.* When Sub-Sequence B of the novel toxin protein CryIE(c) of the present invention is compared with the corresponding region for the related protein CryIE(a), which is not active against *Heliothis virescens,* it is found that the percent similarity is only 63 %.

When CryIE(d) is compared to CryIE(c), the region from amino acid residue 466 to amino acid residue 622 of CryIE(d) differs in only two amino acids. CryIE(d) has a proline at position 482 and a Isoleucine at position 506, compared to a threonine and methionine, respectively, for CrylE(c). These differences do not affect the percent similarity so that Sub-Sequence B of CrylE(d) is also 86% similar to the corresponding region of the CrylA(a) protein.

The present invention is intended to cover mutants and recombinant or genetically engineered derivatives of the CrylE(c) gene (SEQ ID NO:1) or CrylE(d) gene [SEQ ID NO: 7]. For example, it is well known that Cryl-type proteins must be proteolytically cleaved into a toxic core fragment that is the actual toxic molecule [Höfte and Whiteley, Microbiol Rev 53: 242-255 (1989). Hence, a truncated DNA sequence which encodes for the toxic core fragment of the novel toxin protein CryIE(c) and CryIE(d) is considered to be within the scope of the present invention. In addition, a recombinant toxin protein may be constructed using methods known in the art which contain at least one of the sub-sequences conferring activity against *Heliothis virescens* as indicated for CryIE(c) [see SEQ ID NOS: 3 to 4] or the related gene CryIE(d)[see SEQ ID NOS: 9 to 10]. Such a recombinant protein would have activity against *Heliothis virescens,* in addition to its native properties. Mutants of the CryIE(c) and the CryIE(d) genes of the present invention are also encompassed by the present invention. For example, individual nucleotides may be altered, either by natural processes or by site-directed mutagenesis, which in turn creates a change in the amino acid sequence of the encoded protein. In addition, the DNA sequence of either the full-length or truncated form of the genes may be altered such that their expression is optimized for plants, where the codons are chosen so as to produce a proteins having the same or similar amino acid sequence as that encoded by the native form of the CryIE(c) or the CryIE(d) genes. Other modifications of the novel toxin gene that yield a protein with insecticidal properties essentially the same as those of the CryIE(c) protein (SEQ ID NO:2) or CryIE(d) [SEQ ID NO: 8] are also encompassed by the present invention.

The CryIE(c) gene [SEQ ID NO:1] and CryIE(d) gene [SEQ ID NO: 7] or the sub-sequences of the gene which confer specificity against *Heliothis virescens,* are also useful as a DNA hybridization probe, for discovering similar or closely related Cryl-type or genes active against *Heliothis virescens* in other *Bacillus thuringiensis* strains. The novel genes, or portions or derivatives thereof, can be labeled for use as a hybridization probe, e.g., with a radioactive label, using conventional procedures. The labeled DNA hybridization probe may then be used in the manner described in the Example 1.

The utility of the novel toxin genes present in a recombinant strain of *Bacillus thuringiensis* is illustrated in Examples 3 to 4. It should also be recognized that the isolated novel toxin gene of the present invention can be transferred into any microbial host and confer their insecticidal properties upon that host. Alternate hosts for the novel toxin gene of the present invention can be selected as suitable for cloning purposes, for purposes of characterizing the form and function of the gene or encoded protein, for use as a fermentation host to increase production of the toxin protein, for purposes of delivering at least one of the toxin proteins more effectively to the target insect pest, or introduction of the novel toxin gene into insect pathogens such as baculovirus [a nuclear polyhedrosis virus, eg [*Autographica californica*] to improve their effectiveness.

The novel toxin genes or recombinant forms thereof can be transformed into such alternate hosts using a variety of art recognized methods. One such preferred method is electroporation of microbial cells, as described, for example, by the method of Dower (U.S. Patent No. 5,186,800). Another preferred method is that of Schurter et al. (Mol. Gen. Genet. 218: 177-181 (1989)), which is also disclosed in EP-A 0 342 633 which is incorporated herein in its entirety.

It is envisioned that said alternate host would be applied to the environment or plants or animals for insect control. Microorganism hosts may be selected which are known to occupy the "phytosphere" (phylloplane, phyllosphere, rhizosphere, and/or rhizoplana) of one or more crops of interest. These microorganisms are selected so as to be capable of successfully competing in the particular environment with the wild-type microorganisms, provide for stable maintenance and expression of the gene expressing the polypeptide pesticide, and, desirably, provide for improved protection of the pesticide from environmental degradation and inactivation.

Such microorganisms include bacteria, algae, and fungi. Of particular interest are microorganisms, such as bacteria, e.g., *Bacillus, Caulobacter, Agmenellum, Pseudomonas, Erwinia, Serratia, Klebsiella, Xanthomonas, Streptomyces, Rhizobium, Rhodopseudomonas, Methylius, Agrobacterium, Acetobacter, Lactobacillus, Arthrobacter, Azotobacter, Leuconostoc,* and *Alcaligenes*; fungi, particularly yeast, e.g., *Saccharomyces, Cryptococcus, Kluyveromyces, Sporobolomyces, Rhodotorula,* and *Aureobasidium.* Of particular interest are such phytosphere bacterial species as *Bacillus* spp., *Pseudomonas syringae, Pseudomonas fluorescens, Serratia marcescens, Acetobacter xylinum, Agrobacteria, Rhodopseudomonas spheroides, Xanthomonas campestris, Rhizobium melioti, Alcaligenes entrophus, Clavibacterxyli* and *Azotobacter vinlandii,* and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces rosues, S. odorus, Kluyveromyces veronae,* and *Aureobasidium pollulans*. Of particular interest are the pigmented microorganisms.

The present invention further provides an entomocidal composition comprising as an active ingrdient at least one of the novel toxins according to the invention or a recombinant microorganism containing at least on of the novel toxin genes in recombinant form, but especially a recombinant *Bacillus thuringiensis* strain containing at least one of the novel toxin genes in recombinant form, or a derivative or mutant thereof, together with an agricultural adjuvant such as a carrier, diluent, surfactant or application-promoting adjuvant. The composition may also contain a further biologically active compound. The said compound can be both a fertilizer or micronutrient donor or other preparations that influence plant growth. It can also be a selective herbicide, insecticide, fungicide, bactericide, nematicide, molluscide or mixtures of several of these preparations, if desired, together with further agriculturally acceptable carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers

The composition may comprise from 0.1 to 99% by weight of the active ingredient, from 1 to 99.9% by weight of a solid or liquid adjuvant, and from 0 to 25% by weight of a surfactant. The acitve ingredient comprising at least one of the novel toxins according to the invention or a recombinant microorganism containing at least one of the novel toxin genes in recombinant form, but especially a recombinant *Bacillus thuringiensis* strain containing at least one of the novel toxin genes in recombinant form, or a derivative or mutant thereof, or the composition containing the said acitve ingredient, may be administered to the plants or crops to be protected together with certain other insecticides or chemicals (1993 Crop Protection Chemicals Reference, Chemical and Pharmaceutical Press, Canada) without loss of potency. It is compatible with most other commonly used agricultural spray materials but should not be used in extremely alkaline spray solutions. It may be administered as a dust, a suspension, a wettable powder or in any other material form suitable for agricultural application.

The invention further provides methods for for controlling or inhibiting of insect pests by applying an active ingredient comprising at least one of the novel toxins according to the invention or a recombinant microorganism containing at least one of the novel toxin gene in recombinant form or a composition comprising the said active ingredient to (a) an environment in which the insect pest may occur, (b) a plant or plant part in order to protect said plant or plant part from damage caused by an insect pest, or (c) seed in order to protect a plant which develops from said seed from damage caused by an insect pest.

A preferred method of application in the area of plant protection is application to the foliage of the plants (foliar application), with the number of applications and the rate of application depending on the plant to be protected and the risk of infestation by the pest in question. However, the active ingredient may also penetrate the plants through the roots (systemic action) if the locus of the plants is impregnated with a liquid formulation or if the active ingredient is incorporated in solid form into the locus of the plants, for example into the soil, e.g. in granular form (soil application). In paddy rice crops, such granules may be applied in metered amounts to the flooded rice field.

The compositions according to the invention are also suitable for protecting plant propagating material, e.g. seed, such as fruit, tubers or grains, or plant cuttings, from insect pests. The propagation material can be treated with the formulation before planting: seed, for example, can be dressed before being sown. The acitve ingredient of the invention can also be applied to grains (coating), either by impregnating the grains with a liquid formulation or by coating them with a solid formulation. The formulation can also be applied to the planting site when the propagating material is being planted, for example to the seed furrow during sowing. The invention relates also to those methods of treating plant propagation material and to the plant propagation material thus treated.

The compositions according to the invention comprising as an active ingredient a recombinant microorganism containing at least one of the novel toxin genes in recombinant form, but especially a recombinant *Bacillus thuringiensis* strain containing at leat one of the novel toxin genes in recombinant form, or a derivative or mutant thereof may be applied in any method known for treatment of seed or soil with bacterial strains. For example, see US Patent No.4,863,866. The strains are effective for biocontrol even if the microorganism is not living. Preferred is, however, the application of the living microorganism.

Target crops to be protected within the scope of the present invention comprise, e.g., the following species of plants:
cereals (wheat, barley, rye, oats, rice, sorghum and related crops), beet (sugar beet and fodder beet), forage grasses (orchardgrass, fescue, and the like), drupes, pomes and soft fruit (apples, pears, plums, peaches, almonds, cherries, strawberries, raspberries and blackberries), leguminous plants (beans, lentils, peas, soybeans), oil plants (rape, mustard, poppy, olives, sunflowers, coconuts, castor oil plants, cocoa beans, groundnuts), cucumber plants (cucumber, marrows, melons) fiber plants (cotton, flax, hemp, jute), citrus fruit (oranges, lemons, grapefruit, mandarins), vegetables (spinach, lettuce, asparagus, cabbages and other Brassicae, onions, tomatoes, potatoes, paprika), lauraceae (avocados, carrots, cinnamon, camphor), deciduous trees and conifers (e.g. linden-trees, yew-trees, oak-trees, alders, poplars, birch-trees, firs, larches, pines), or plants such as maize, tobacco, nuts, coffee, sugar cane, tea, vines, hops, bananas and natural rubber plants, as well as ornamentals (including composites).

A recombinant *Bacillus thuringiensis* strain containing at least one of the novel gene in recombinant form is normally applied in the form of entomocidal compositions and can be applied to the crop area or plant to be treated, simultaneously or in succession, with further biologically active compounds. These compounds may be both fertilizers or micronutrient donors or other preparations that influence plant growth. They may also be selective herbicides, insecticides, fungicides, bactericides, nematicides, molluscicides or mixtures of several of these preparations, if desired together with further carriers, surfactants or application-promoting adjuvants customarily employed in the art of formulation.

The active ingredient according to the invention may be used in unmodified form or together with any suitable agriculturally acceptable carrier. Such carriers are adjuvants conventionally employed in the art of agricultural formulation, and are therefore formulated in known manner to emulsifiable concentrates, coatable pastes, directly sprayable or dilutable solutions, dilute emulsions, wettable powders, soluble powders, dusts, granulates, and also encapsulations, for example, in polymer substances. Like the nature of the compositions, the methods of application, such as spraying, atomizing, dusting, scattering or pouring, are chosen in accordance with the intended objective and the prevailing circumstances. Advantageous rates of application are normally from about 50 g to about 5 kg of active ingredient (a.i.) per hectare ("ha", approximately 2.471 acres), preferably from about 100 g to about 2kg a.i./ha. Important rates of application are about 200 g to about 1 kg a.i./ha and 200g to 500g a.i./ha.
For seed dressing advantageous application rates are 0.5 g to 1000 g a.i.per 100 kg seed, preferably 3 g to 100 g a.i. per 100 kg seed or 10 g to 50 g a.i.per 100 kg seed.

Suitable carriers and adjuvants can be solid or liquid and correspond to the substances ordinarily employed in formulation technology, e.g. natural or regenerated mineral substances, solvents, dispersants, wetting agents, tackifiers, binders or fertilizers. The formulations, i.e, the entomocidal compositions, preparations or mixtures containing the recombinant *Bacillus thuringiensis* strain containing the novel gene in recombinant form as an active ingredient or combinations thereof with other active ingredients, and, where appropriate, a solid or liquid adjuvant, are prepared in known manner, e.g., by homogeneously mixing and/or grinding the active ingredients with extenders, e.g., solvents, solid carriers, and in some cases surface-active compounds (surfactants).

Suitable solvents are: aromatic hydrocarbons, preferably the fractions containing 8 to 12 carbon atoms, e.g. xylene mixtures or substituted naphthalenes, phthalates such as dibutyl phthalate or dioctyl phthalate, aliphatic hydrocarbons such as cyclohexane or paraffins, alcohols and glycols and their ethers and esters, such as ethanol, ethylene glycol monomethyl or monoethyl ether, ketones such as cyclohexanone, strongly polar solvents such as N-methyl-2-pyrrolidone, dimethylsulfoxide or dimethylformamide, as well as vegetable oils or epoxidised vegetable oils such as epoxidised coconut oil or soybean oil; or water.

The solid carriers used, e.g., for dusts and dispersible powders, are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverized plant residues.

Depending on the nature of the active ingredients to be formulated, suitable surface-active compounds are non-ionic, cationic and/or anionic surfactants having good emulsifying, dispersing and wetting properties. The term "surfactants" will also be understood as comprising mixtures of surfactants. Suitable anionic surfactants can be both water-soluble soaps and
water-soluble synthetic surface-active compounds. Suitable soaps are the alkali metal salts, alkaline earth metal salts or unsubstituted or substituted ammonium salts of higher fatty acids (C sub 10 -C sub 22), e.g. the sodium or potassium salts of oleic or stearic acid, or of natural fatty acid mixtures which can be obtained, e.g. from coconut oil or tallow oil. Further suitable surfactants are also the fatty acid methyltaurin salts as well as modified and unmodified phospholipids.

More frequently, however, so-called synthetic surfactants are used, especially fatty sulfonates, fatty sulfates, sulfonated benzimidazole derivatives or alkylarylsulfonates. The fatty sulfonates or sulfates are usually in the forms of alkali metal salts, alkaline earth metal salts or unsubstituted or substituted ammonium salts and generally contain a C sub 8 -C sub 22 alkyl radical which also includes the alkyl moiety of acyl radicals, e.g. the sodium or calcium salt of lignosulfonic acid, of dodecylsulfate, or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing about 8 to 22 carbon atoms. Examples of alkylarylsulfonates are the sodium, calcium or triethanolamine salts of dodecylbenzenesulfonic acid, dibutylnaphthalenesulfonic acid, or of a naphthalenesulfonic acid/formaldehyde condensation product. Also suitable are corresponding phosphates, e.g. salts of the phosphoric acid ester of an adduct of p-nonylphenol with 4 to 14 moles of ethylene oxide. Non-ionic surfactant are preferably polyglycol ether derivatives of aliphatic or cycloaliphatic alcohols, or saturated or unsaturated fatty acids and alkylphenols, said derivatives containing 3 to 30 glycol ether groups and 8 to 20 carbon atoms in the (aliphatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediaminopolypropylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit. Representative examples of non-ionic surfactants are nonylphenolpolyethoxyethanols, castor oil polyglycol ethers, polypropylene/polyethylene oxide adducts, tributylphenoxypolyethoxyethanol, polyethylene glycol and octylphenoxypolyethoxyethanol. Fatty acid esters of polyoxyethylene sorbitan, such as polyoxyethylene sorbitan trioleate, are also suitable non-ionic surfactants.

Cationic surfactants are preferably quaternary ammonium salts which contain, as N-substituent, at least one C sub 8 -C sub 22 alkyl radical and, as further substituents, lower unsubstituted or halogenated alkyl, benzyl or hydroxyl-lower alkyl radicals. The salts are preferably in the form of halides, methylsulfates or ethylsulfates, e.g., stearyltrimethylammonium chloride or benzyldi-(2-chloroethyl)ethylammonium bromide.

The surfactants customarily employed in the art of formulation are described, e.g., in "McCutcheon's Detergents and Emulsifiers Annual", MC Publishing Corp. Ridgewood, N.J., 1979; Dr. Helmut Stache, "Tensid Taschenbuch" (Handbook of Surfactants), Carl Hanser Verlag, MunichNienna.

Another particularly preferred characteristic of an entomocidal composition of the present invention is the persistence of the active ingredient when applied to plants and soil. Possible causes for loss of activity include inactivation by ultra-violet light, heat, leaf exudates and pH. For example, at high pH, particularly in the presence of reductant, δ-endotoxin crystals are solubilized and thus become more accessible to proteolytic inactivation. High leaf pH might also be important, particularly where the leaf surface can be in the range of pH 8-10. Formulation of an entomocidal composition of the present invention can address these problems by either including additives to help prevent loss of the active ingredient or encapsulating the material in such a way that the active ingredient is protected from inactivation. Encapsulation can be accomplished chemically (McGuire and Shasha, J Econ Entomol 85: 1425-1433, 1992) or biologically (Barnes and Cummings, 1986; EP-A 0 192 319). Chemical encapsulation involves a process in which the active ingredient is coated with a polymer while biological encapsulation involves the expression of the δ-endotoxin genes in a microbe. For biological encapsulation, the intact microbe containing the δ-endotoxin protein is used as the active ingredient in the formulation. The addition of UV protectants might effectively reduce irradiation damage. Inactivation due to heat could also be controlled by including an appropriate additive.

Preferred within the present application are formulations comprising living microorganisms as active ingredient either in form of the vegetative cell or more preferable in form of spores, if available. Suitable formulations may consist, for example, of polymer gels which are crosslinked with polyvalent cations and comprise these microorganisms. This is described, for example, by D.R. Fravel et al. in Phytopathology, Vol. 75, No. 7, 774-777, 1985 for alginate as the polymer material. It is also known from this publication that carrier materials can be co-used. These formulations are as a rule prepared by mixing solutions of naturally occurring or synthetic gel-forming polymers, for example alginates, and aqueous salt solutions of polyvalent metal ions such that individual droplets form, it being possible for the microorganisms to be suspended in one of the two or in both reaction solutions. Gel formation starts with the mixing in drop form. Subsequent drying of these gel particles is possible. This process is called ionotropic gelling. Depending on the degree of drying, compact and hard particles of polymers which are structurally crosslinked via polyvalent cations and comprise the microorganisms and a carrier present predominantly uniformly distributed are formed. The size of the particles can be up to 5 mm.

Compositions based on partly crosslinked polysaccharides which, in addition to a microorganism, for example, can also comprise finely divided silicic acid as the carrier material, crosslinking taking place, for example, via Ca⁺⁺ ions, are described in EP-A1-0 097 571. The compositions have a water activity of not more than 0.3. W.J. Cornick et al. describe in a review article [New Directions in Biological Control: Alternatives for Suppressing Agricultural Pests and Diseases, pages 345-372, Alan R. Liss, Inc. (1990)] various formulation systems, granules with vermiculite as the carrier and compact alginate beads prepared by the ionotropic gelling process being mentioned. Such compositions are also disclosed by D.R.Fravel in Pesticide Formulations and Application Systems: 11th Volume, ASTM STP 1112 American Society for Testing and Materials, Philadelphia, 1992, pages 173 to 179 and can be used to formulate the recombinant microorganisms according to the invention.

The entomocidal compositions of the invention usually contain from about 0.1 to about 99%, preferably about 0.1 to about 95%, and most preferably from about 3 to about 90% of the active ingredient, from about 1 to about 99.9%, preferably from about 1 to about 99%, and most preferably from about 5 to about 95% of a solid or liquid adjuvant, and from about 0 to about 25%, preferably about 0.1 to about 25%, and most preferably from about 0.1 to about 20% of a surfactant.

In a preferred embodiment of the invention the entomocidal compositions usually contain 0.1 to 99%, preferably 0.1 to 95%, of a recombinant *Bacillus thuringiensis* strain containing at least one of the novel genes in recombinant form, or combination thereof with other active ingredients, 1 to 99.9% of a solid or liquid adjuvant, and 0 to 25%, preferably 0.1 to 20%, of a surfactant.

Whereas commercial products are preferably formulated as concentrates, the end user will normally employ dilute formulations of substantially lower concentration. The entomocidal compositions may also contain further ingredients, such as stabilizers, antifoams, viscosity regulators, binders, tackifiers as well as fertilizers or other active ingredients in order to obtain special effects.

A further object ot the invention relates to transgenic plants comprising at least one of the novel toxin genes according to the invention. A host plant expressing the novel toxin genes of the invention will have enhanced resistance to insect attack and will be thus better equipped to withstand crop losses associated with such attack.

By plant is meant any plant species which can be genetically transformed by methods known in the art. Methods known in the art for plant transformation are disucssed below. Host plants include, but are not limited to, those speicies previously listed as target crops.

It has been discovered that the codon usage of a native *Bacillus thuringiensis* toxin gene is significantly different from that which is typical of a plant gene. In particular, the codon usage of a native *Bacillus thuringiensis* gene is very different from that of a maize gene. As a result, the mRNA from this gene may not be efficiently utilized. Codon usage might influence the expression of genes at the level of translation or transcription or mRNA processing. To optimize a toxin gene for expression in plants, for example in maize, the codon usage is optimized by using the codons which are most preferred in maize (maize preferred codons) in the synthesis of a synthetic gene which encodes the same protein as found for the native toxin gene sequence. The optimized maize preferred codon usage is effective for expression of high levels of the *Bt* insecticidal protein. Further details for constructing maize-optimized synthetic toxin genes can be found in WO 93/07278, herein incorporated by reference in its entirety.

Toxin genes derived from microorganisms may also differ from plant genes. Plant genes differ from genes found in microorganisms in that their transcribed RNA does not possess defined ribosome binding site sequence adjacent to the initiating methionine. Consequently, microbial genes can be enhanced by the inclusion of a eukaryotic consensus translation initiator at the ATG. Clontech (1993/1994 catalog, page 210) has suggested the sequence GTCGACCATGGTC (SEQ ID NO:5) as a consensus translation initiator for the expression of the *E. coli uidA* gene in plants. Further, Joshi (Nucl Acids Res 15: 6643-6653 (1987)) has compared many plant sequences adjacent to the ATG and suggests the consensus TAAACAATGGCT (SEQ ID NO:6). In situations where difficulties are encountered in the expression of microbial ORFs in plants, inclusion of one of these sequences at the initiating ATG may improve translation. In such cases the last three nucleotides of the consensus may not be appropriate for inclusion in the modified sequence due to their modification of the second AA residue. Preferred sequences adjacent to the initiating methionine may differ between different plant species. By surveying the sequence of maize genes present in the GenBank/EMBL database it can be discerned which nucleotides adjacent to the ATG should be modified to enhance translation of the toxin gene introduced into maize.

In addition, it has been shown that removal of illegitimate splice sites can enhance expression and stability of introduced genes. Genes cloned from non-plant sources and not optimized for expression in plants may contain motifs which can be recognized in plants as 5' or 3' splice sites. Consequently, the transcription process can be prematurely terminated, generating truncated or deleted mRNA. The toxin genes can be engineered to remove these illegitimate splice sites using the techniques well known in the art.

It is well known that many δ-endotoxin proteins from *Bacillus thuringiensis* are actually expressed as protoxins. These protoxins are solubilized in the alkaline environment of the insect gut and are proteolytically converted by proteases into a toxic core fragment (Höfte and Whiteley, Microbiol. Rev. 53: 242-255 (1989)). For δ-endotoxin proteins of the Cryl class, the toxic core fragment is localized in the N-terminal half of the protoxin. It is within the scope of the present invention that genes encoding either the full-length protoxin form or the truncated toxic core fragment of the novel toxin protein can be used in plant transformation vectors to confer insecticidal properties upon the host plant.

The recombinant DNA molecules can be introduced into the plant cell in a number of art-recognized ways. Those skilled in the art will appreciate that the choice of method might depend on the type of plant, i.e. monocot or dicot, targeted for transformation. Suitable methods of transforming plant cells include microinjection (Crossway et al., BioTechniques 4:320-334 (1986)), electroporation (Riggs et al, Proc. Natl. Acad. Sci. USA 83:5602-5606 (1986), *Agrobacterium*-mediated transformation (Hinchee et al., Biotechnology 6:915-921 (1988)), direct gene transfer (Paszkowski et al., EMBO J. 3:2717-2722 (1984)), and ballistic particle acceleration using devices available from Agracetus, Inc., Madison, Wisconsin and Dupont, Inc., Wilmington, Delaware (see, for example, Sanford et al., U.S. Patent 4,945,050; and McCabe et al., Biotechnology 6:923-926 (1988)). See also, Weissinger et al., Annual Rev. Genet. 22:421-477 (1988); Sanford et al., Particulate Science and Technology 5:27-37 91987)(onion); Christou et al., Plant Physiol. 87:671-674 (1988)(soybean); McCabe et al., Bio/Technology 6:923-926 (1988)(soybean); Datta et al., Bio/Technology 8:736-740 (1990)(rice); Klein et al., Proc. Natl. Acad. Sci. USA, 85:4305-4309 (1988)(maize); Klein et al., Bio/Technology 6:559-563 (1988)(maize); Klein et al., Plant Physiol. 91:440-444 (1988)(maize); Fromm et al., Bio/Technology 8:833-839 (1990); and Gordon-Kamm et al., Plant Cell 2:603-618 (1990)(maize); Svab et al. Proc. Natl. Acad. Sci. USA 87: 8526-8530 (1990) (tobacco chloroplast); Koziel *et al*. (Biotechnology 11: 194-200 (1993)) (maize); Shimamoto *et al*. Nature 338: 274-277 (1989) (rice); Christou *et al*. Biotechnology 9: 957-962 (1991) (rice); European Patent Application EP 0 332 581 (orchardgrass and other *Pooideae*); Vasil *et al*. (Biotechnology 11: 1553-1558 (1993) (wheat); Weeks *et al*. (Plant Physiol. 102: 1077-1084 (1993) (wheat); Wan *et al* (Plant Physiol 104: 37-48 (1994) (barley); Umbeck *et al,* (Bio/Technology 5: 263-266 (1987) (cotton).

One particularly preferred set of embodiments for the introduction of recombinant DNA molecules into maize by microprojectile bombardment can be found in WO 93/07278, herein incorporated by reference in its entirety. An additional preferred embodiment is the protoplast transformation method for maize as disclosed in European Patent Application EP 0 292 435, hereby incorporated by reference in its entirety.

Further comprised within the scope of the present invention are transgenic plants, in particular transgenic fertile plants transformed by means of the aforedescribed processes and their asexual and/or sexual progeny, which still comprises an isolated DNA molecule encoding a toxin protein of the CrylE type according to the invention, which is active against *Heliothis* species but does not exhibit an activity against *Spodoptera exigua.*

The transgenic plant according to the invention may be a dicotyledonous or a monocotyledonous plant. Preferred are monocotyledonous plants of the *Graminaceae* family involving *Lolium, Zea, Triticum, Triticale, Sorghum, Saccharum, Bromus, Oryzae, Avena, Hordeum, Secale* and *Setaria* plants.

Especially preferred are transgenic maize, wheat, barley, sorghum, rye, oats, turf grasses and rice.

Among the dicotyledonous plants soybean, cotton, tobacco, sugar beet, oilseed rape, and sunflower are especially preferred herein.

The expression 'progeny' is understood to embrace both, "asexually" and "sexually" generated progeny of transgenic plants. This definition is also meant to include all mutants and variants obtainable by means of known processes, such as for example cell fusion or mutant selection and which still exhibit the characteristic properties of the initial transformed plant, together with all crossing and fusion products of the transformed plant material.

Another object of the invention concerns the proliferation material of transgenic plants.

The proliferation material of transgenic plants is defined relative to the invention as any plant material that may be propagated sexually or asexually *in vivo* or *in vitro.* Particularly preferred within the scope of the present invention are protoplasts, cells, calli, tissues, organs, seeds, embryos, pollen, egg cells, zygotes, together with any other propagating material obtained from transgenic plants.

Parts of plants, such as for example flowers, stems, fruits, leaves, roots originating in transgenic plants or their progeny previously transformed by means of the process of the invention and therefore consisting at least in part of transgenic cells, are also an object of the present invention.

Transformation of plants can be undertaken with a single DNA species or multiple DNA species (*i*.*e*. co-transformation) and both these techniques are suitable for use with the novel toxin genes of the present invention.

Methods using either a form of direct gene transfer or *Agrobacterium*-mediated transfer usually, but not necessarily, are undertaken with a selectable marker which may provide resistance to an antibiotic (kanamycin, hygromycin or methotrexate) or a herbicide (phosphinothricin). The choice of selectable marker for plant transformation is not, however, critical to the invention.

Numerous transformation vectors are available for plant transformation, and the genes of this invention can be used in conjunction with any such vectors. The selection of vector for use will depend upon the preferred transformation technique and the target species for transformation. For certain target species, different antibiotic or herbicide selection markers may be preferred. Selection markers used routinely in transformation include the *nptll* gene which confers resistance to kanamycin and related antibiotics (Messing & Vierra, Gene 19: 259-268 (1982); Bevan *et al*., Nature 304:184-187 (1983)), the bargene which confers resistance to the herbicide phosphinothricin (White *et al*., Nucl Acids Res 18: 1062 (1990), Spencer *et al*. Theor Appl Genet 79: 625-631(1990)), the *hph* gene which confers resistance to the antibiotic hygromycin (Blochinger & Diggelmann, Mol Cell Biol 4: 2929-2931, 1984), and the *dhfr* gene, which confers resistance to methotrexate (Bourouis *et al*., EMBO J. 2: 1099-1104 (1983).

Many vectors are available for transformation using *Agrobacterium tumefaciens*. These typically carry at least one T-DNA border sequence and include vectors such as pBIN19 (Bevan, Nucl. Acids Res. (1984)). In one preferred embodiment, the novel toxin gene of the present invention may be inserted into either of the binary vectors pCIB200 and pCIB2001 for use with *Agrobacterium.* These vector cassettes for *Agrobacterium-mediated* transformation can be constructed in the following manner. pTJS75kan was created by *Narl* digestion of pTJS75 (Schmidhauser & Helinski, J Bacteriol. 164: 446-455 (1985)) allowing excision of the tetracycline-resistance gene, followed by insertion of an *Accl* fragment from pUC4K carrying an NPTII (Messing & Vierra, Gene 19: 259-268 (1982); Bevan *et al*., Nature 304: 184-187 (1983); McBride *et al.,* Plant Molecular Biology 14: 266-276 (1990)). *Xhol* linkers were ligated to the *EcoRV* fragment of pCIB7 which contains the left and right T-DNA borders, a plant selectable *nos*/*nptll* chimeric gene and the pUC polylinker (Rothstein *et al.,* Gene 53: 153-161 (1987)), and the *XhoI*-digested fragment was cloned into *SalI*-digested pTJS75kan to create pCIB200 (see also EP 0 332 104, example 19). pCIB200 contains the following unique polylinker restriction sites: *EcoRl, Sstl, KpnI, BglII, Xbal,* and *Sall. pCIB2001* is a derivative of pCIB200 which is created by the insertion into the polylinker of additional restriction sites. Unique restriction sites in the polylinker of pCIB2001 are *EcoRl, Sstl, Kpnl, Bglll, Xbal, Sall, Mlul, Bcll, Avrll, Apal, Hpal,* and *Stul.* pCIB2001, in addition to containing these unique restriction sites also has plant and bacterial kanamycin selection, left and right T-DNA borders for *Agrobacterium*-mediated transformation, the RK2-derived *trfA* function for mobilization between *E. coli* and other hosts, and the *OriT*and *OriV* functions also from RK2. The pCIB2001 polylinker is suitable for the cloning of plant expression cassettes containing their own regulatory signals.

An additional vector useful for *Agrobacterium*-mediated transformation is the binary vector pCIB10 which contains a gene encoding kanamycin resistance for selection in plants, T-DNA right and left border sequences and incorporates sequences from the wide host-range plasmid pRK252 allowing it to replicate in both *E. coli* and *Agrobacterium.* Its construction is described by Rothstein *et al.* (Gene 53: 153-161 (1987)). Various derivatives of pCIB10 have been constructed which incorporate the gene for hygromycin B phosphotransferase described by Gritz *et al.* (Gene 25: 179-188 (1983)). These derivatives enable selection of transgenic plant cells on hygromycin only (pCIB743), or hygromycin and kanamycin (pCIB715, pCIB717).

Other transformation techniques which do not rely on *Agrobacterium,* the so-called direct gene transfer methods, are also useful for the introduction of the novel toxin gene of the present invention, including transformation by microprojectile bombardment, protoplast uptake (*e.g.* PEG and electroporation) and microinjection. The choice of vector for these methods depends largely on the preferred selection for the species being transformed.

One such vector useful for direct gene transfer techniques in combination with selection by the herbicide Basta (or phosphinothricin) is pCIB3064. This vector is based on the plasmid pCIB246, which comprises the CaMV 35S promoter in operational fusion to the *E. coli* GUS gene and the CaMV 35S transcriptional terminator and is described in the PCT published application WO 93/07278, herein incorporated by reference. The gene for providing resistance to phosphinothricin is the *bar* gene from *Streptomyces hygroscopicus* (Thompson *et al*. EMBO J 6: 2519-2523 (1987)). This vector is suitable for the cloning of plant expression cassettes containing their own regulatory signals.

An additional transformation vector is pSOG35 which utilizes the *E. coli* gene dihydrofolate reductase (DHFR) as a selectable marker conferring resistance to methotrexate. PCR was used to amplify the 35S promoter (~800 bp), intron 6 from the maize Adh1 gene (~550 bp) [Lou et al, Plant J 3: 393-403, 1993; Dennis et al, Nucl Acids Res 12: 3983-4000, 1984] and 18 bp of the GUS untranslated leader sequence from pSOG10 [see also Jefferson *et al*, Proc Natl Acad Sci USA 83: 8447-8451 (1986)]. A 250 bp fragment encoding the *E*. *coli* dihydrofolate reductase type II gene was also amplified by PCR and these two PCR fragments were assembled with a *Sacl-Pstl* fragment from pB1221 (Clontech) which comprised the pUC19 vector backbone and the nopaline synthase terminator. Assembly of these fragments generated pSOG19 which contains the 35S promoter in fusion with the intron 6 sequence, the GUS leader, the DHFR gene and the nopaline synthase terminator. Replacement of the GUS leader in pSOG19 with the leader sequence from Maize Chlorotic Mottle Virus *check* (MCMV) generated the vector pSOG35. pSOG19 and pSOG35 carry the pUC gene for ampicillin resistance and have *Hindlll, Sphl, Pstl* and *EcoRl* sites available for the cloning of foreign sequences.

The novel toxin genes of the present invention, either as their native sequence or as optimized synthetic sequences as described above, can be operably fused to a variety of promoters for expression in plants including constitutive, inducible, temporally regulated, developmentally regulated, chemically regulated, tissue-preferred and tissue-specific promoters to prepare recombinant DNA molecules, i.e., chimeric genes. Preferred constitutive promoters include the CaMV 35S and 19S promoters [Fraley *et al*, US Patent No 5,352, 605., issued October 04, 1994]. An additionally preferred promoter is derived from any one of several of the actin genes, which are known to be expressed in most cell types. The promoter expression cassettes described by McElroy *et al*. (Mol. Gen. Genet. 231: 150-160 (1991)) can be easily modified for the expression of the novel toxin gene and are particularly suitable for use in monocotyledonous hosts.

Yet another preferred constitutive promoter is derived from ubiquitin, which is another gene product known to accumulate in many cell types. The ubiquitin promoter has been cloned from several species for use in transgenic plants (*e.g.* sunflower - Binet *et al.* Plant Science 79: 87-94 (1991), maize - Christensen *et al*. Plant Molec. Biol. 12: 619-632 (1989)). The maize ubiquitin promoter has been developed in transgenic monocot systems and its sequence and vectors constructed for monocot transformation are disclosed in the patent publication EP 0 342 926. The ubiquitin promoter is suitable for the expression of the novel toxin gene in transgenic plants, especially monocotyledons.

Tissue-specific or tissue-preferential promoters useful for the expression of the novel toxin gene in plants, particularly maize, are those which direct expression in root, pith, leaf or pollen. Such promoters are disclosed in WO 93/07278, herein incorporated by reference in its entirety. Chemically inducible promoters useful for directing the expression of the novel toxin gene in plants are disclosed in EP-A 0 332 104, herein incorporated by reference in its entirety.

In addition to promoters, a variety of transcriptional terminators are also available for use in chimeric gene construction using the novel toxin gene of the present invention. Transcriptional terminators are responsible for the termination of transcription beyond the transgene and its correct polyadenylation. Appropriate transcriptional terminators and those which are known to function in plants include the CaMV 35S terminator, the *tml* terminator, the nopaline synthase terminator, the pea *rbcS* E9 terminator and others known in the art. These can be used in both monocotyledons and dicotyledons.

Numerous sequences have also been found to enhance gene expression from within the transcriptional unit and these sequences can be used in conjunction with the novel toxin gene of this invention to increase their expression in transgenic plants.

Various intron sequences have been shown to enhance expression, particularly in monocotyledonous cells. For example, the introns of the maize *Adh1* gene have been found to significantly enhance the expression of the wild-type gene under its cognate promoter when introduced into maize cells (Callis *et al*., Genes Develop 1: 1183-1200 (1987)). Intron sequences have been routinely incorporated into plant transformation vectors, typically within the non-translated leader.

A number of non-translated leader sequences derived from viruses are also known to enhance expression, and these are particularly effective in dicotyledonous cells. Specifically, leader sequences from Tobacco Mosaic Virus (TMV, the "Ω-sequence"), Maize Chlorotic Mottle Virus (MCMV), and Alfalfa Mosaic Virus (AMV) have been shown to be effective in enhancing expression (*e.g.* Gallie *et al*. Nucl. Acids Res. 15: 8693-8711 (1987); Skuzeski *et al*. Plant Molec. Biol. 15; 65-79 (1990))

### BRIEF DESCRIPTION OF THE FIGURE

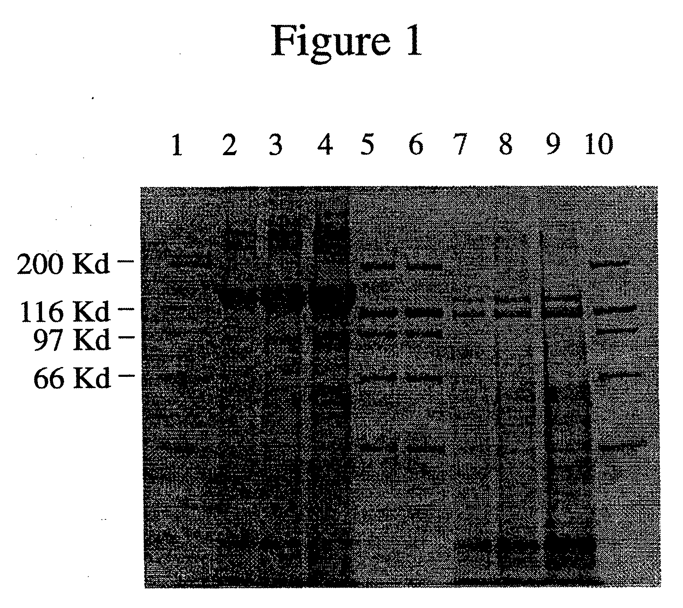

Figure 1. SDS-PAGE analysis of the CryIE(c) and CryIE(d) proteins individually expressed in the recombinant *Bacillus thuringiensis* strains CGB311 and CGB313, respectively.

Lane 1, molecular weight markers; lane 2, CBG311 5 I; lane 3, CGB313 10 I; lane 4, CGB311 20 I; 5,molecular weight markers; 6, molecular weight markers; lane 7, CBG313 5 I; lane 8, CGB313 10 I; lane 9, CGB313 20 I; 10 ,molecular weight markers.

### EXAMPLES

The following examples further describe the materials and methods used in carrying out the invention and the subsequent results. They are offered by way of illustration, and their recitation should not be considered as a limitation of the claimed invention.

### Formulation Examples

The active ingredient used in following formulation examples are the recombinant strains of *Bacillus thuringiensis* designated CGB311 and CGB313, respectively containing pCIB5618 and pCIB5621, respectively and expressing the novel toxin genes CryIE(c) and CryIE(d) respectively. Both CGB313 and CGB311 were deposited on June 1, 1994 with the Agricultural Research Service, Patent Culture Collection (NRRL), Northern Regional Research Center, 1815 North University Street, Peoria, Illinois 61604, U.S.A. and were assigned accession numbers NRRL B-21274 and NRRL B-21273, respectively.

### A1. Wettable powders

| | a) | b) | c) |
|---|---|---|---|
| *Bacillus thuringiensis* spores | 25% | 50% | 75% |
| sodium lignosufonate | 5% | 5% | -- |
| sodium laurylsulfate | 3% | -- | 5% |
| sodium diisobutylnaphthalenesulfonate | -- | 6% | 10% |
| octylphenol polyethylene glycol ether | -- | 2% | -- |
| (7-8 moles of ethylene oxid) | | | |
| highly dispersed silicid acid | 5% | 10% | 10% |
| kaolin | 62% | 27% | -- |

The spores are thoroughly mixed with the adjuvants and the mixture is thoroughly ground in a suitable mill, affording wettable powders which can be diluted with water to give suspensions of the desired concentrations.

### A2. Emulsifiable concentrate

| | |
|---|---|
| *Bacillus thuringiensis* spores | 10% |
| octylphenol polyethylene glycol ether (4-5 moles ethylene oxide) | 3% |
| clacium dodecylbenzensulfonate | 3% |
| castor oil polyglycol ether (36 moles of ethylene oxide) | 4% |
| cyclohexanone | 30% |
| xylene mixture | 50% |

Emulsions of any required concentration can be obtained from this concentrate by dilution with water.

### A3. Dusts

| | a) | b) |
|---|---|---|
| *Bacillus thuringiensis* spores | 5% | 8% |
| talcum | 95% | -- |
| kaolin | -- | 92% |

Ready for use dusts are obtained by mixing the active ingredient with the carriers and grinding the mixture in a suitable mill.

### A4. Extruder Granulate

| | |
|---|---|
| *Bacillus thuringiensis* spores | 10% |
| sodium lignosulfonate | 2% |
| carboxymethylcellulose | 1% |
| kaolin | 87% |

The active ingredient or combination is mixed and ground with the adjuvants and the mixture is subsequently moistened with water. The mixture is extruded, granulated and the dried in a stream of air.

### A5. Coated Granule

| | |
|---|---|
| *Bacillus thuringiensis* spores | 3% |
| polyethylene glycol (mol wt 200) | 3% |
| kaolin | 94% |

The active ingredient or combination is uniformly applied in a mixer to the kaolin moistened with polyethylene glycol. Non-dusty coated granulates are obtained in this manner.

### A6. Suspension Concentrate

| | |
|---|---|
| *Bacillus thuringiensis* spores | 40% |
| ethylene glycol | 10% |
| nonylphenol polyethylene glycol ether (15 moles of ethylene oxide) | 6% |
| sodium lignosulfonate | 10% |
| carboxymethylcellulose | 1% |
| 37% aqueous formaldehyde solution | 0.2% |
| silicone oil in the form of a 75% aqueous solution | 0.8% |
| water | 32% |

The active ingredient or combination is intimately mixed with the adjuvants giving a suspension concentrate from which suspensions of any desired concentration can be obtained by dilution with water.

### Example 1: Cloning and Isolation of the Novel Toxin Gene

Total DNA was isolated from an appropriate strain of *Bacillus thuringiensis* var *kurstaki.* For the isolation of CryIE(c), the strain used was CGB316 and for CryIE(d) the strain used was CGB323. A culture of the appropriate strain was grown overnight in L-broth at 25 °C with 150 rpm of shaking in a rotary shaker. The culture was then centrifuged, and resuspended in 8% sucrose, 100 mM Tris pH 8.0, 10 mM EDTA, 50 mM NaCl and 2 mg/ml lysozyme, and incubated for 30 minutes at 37 °C. Fifty µg/ml proteinase K and SDS to a final concentration of 0.2% were then added and the resultant mixture incubated at 50 °C until the solution became very viscous. An equal volume of phenol/chloroform was added to the viscous mixture, vortexed and centrifuged to separate the aqueous and organic phases. The aqueous phase was then mixed with 1 g/ml CsCl and 150 µg/ml ethidium bromide, placed in an 33 ml Nalgene UltraLok tube and centrifuged at 45,000 rpm for 16 hours in a Beckman Ti50 ultracentrifuge rotor. The resultant DNA band was visualized with a UV light source and was removed with a syringe using a 16 gauge needle.
Contaminating ethidium bromide was removed from the DNA sample by isoamyl alcohol extraction. The isolated DNA was precipitated with 2 volumes 100% ethanol and centrifuged. The resulting DNA pellet was washed with 70% ethanol. The DNA pellet was dried and resuspended in 10 mM Tris pH 8.0, 1 mM EDTA.

Fifteen µg of isolated DNA from each strain was digested with 0.1 units Sau3A / µg DNA at 37 °C. At 3, 5 and 10 minutes after addition of restriction enzyme 5 µg samples were removed from the digest mixture, EDTA was added made to a final concentration of 10 mM and placed on ice. An aliquot of each timed digest was loaded on a 0.8% agarose gel utilizing a Tris-borate-EDTA (TBE; Molecular Cloning, A Laboratory Manual, Sambrook et al. eds. Cold Spring Harbor Press: New York (1989)) buffer and run overnight at 25 volts in an IBI model MPH gel electrophoresis system [Kodak, Rochester, New York]. Lambda DNA digested with HindIII was used as molecular weight markers for the gel run. After electrophoresis, DNA fragments in the 6-9 kb range were cut out of the gel. DNA was electroeluted out of the agarose gel slices by placing them in a Nanotrap electroelution trap [ISCO, Lincoln, Nebraska] and utilizing a ISCO Little Blue Tank [ISCO, Lincoln, Nebraska] with a buffer system and current using the procedure described by the supplier of the apparatus. After electroelution, the isolated DNA was precipitated by addition of 1/10 volume 3M sodium acetate pH 4.8, 2.5 volumes 100% ethanol and then centrifuged. The resulting DNA pellet was washed with 70% ethanol and centrifuged. The DNA pellet was dried and resuspended in 10 mM Tris pH 8.0, 1 mM EDTA.

Ligation into pUC19 [New England Biolabs, Beverly, Massachusetts, USA] of the isolated DNA fragments having sizes of 6-9 kb were done using 4 µl of the above DNA solution, 1 µl of a 100 ng/µl solution of pUC19 that was previously digested with Bam HI and treated with calf alkaline phosphatase, 1 µl 10X ligation buffer, 3 µl water and 1 µl containing 3 units T4 ligase. This mixture was incubated at 15 °C overnight. The resulting pUC19-based plasmids containing the inserted DNA fragments isolated from the *Bacillus thuringiensis* strain was transformed into *E. coli* DH5α [Stratagene, LaJolla, California, USA] competent cells. This was accomplished by combining the ligation mixture with 200 µl of bacterial cells, placement on ice for 1-2 hours and subsequent heating at 42 °C for 90 seconds. After treatment the bacterial solution was mixed with 200 µl of SOC medium (Molecular Cloning, A Laboratory Manual, Sambrook et al. eds. Cold Spring Harbor Press: New York (1989)), placed at 37 °C for 45 minutes and plated on L-agar plates containing 100 µg/ml ampicillin to select for transformants. The plates were incubated overnight at 37 °C.

Transformed colonies arising after overnight incubation were subjected to the colony hybridization procedure as described in Molecular Cloning, A Laboratory Manual, Sambrook et al. eds. Cold Spring Harbor Press: New York (1989).
In brief, a 85 mm Nitroplus 2000 filter circle [Micron Separations, Westboro, Massachusetts, USA] was placed on each agar plate containing transformed colonies and then lifted off. After removing the filter, the plates were again placed at 37 °C until transformed colonies were visible again. The filters with the colonies on them were treated to release DNA from the bacterial cells first on Whatman paper saturated with 10% SDS, then 0.5 N NaOH-1.5 M NaCl, then 1.5 M NaCl-0.5 M Tris pH 7.4 for 3 minutes each. The filters were then treated with 2X SSC and the released bacterial DNA was fixed to the filters by UV crosslinking using a Stratalinker (Stratagene) at 0.2 mJoule.

Approximately 6 plates with 100-200 colonies/plate were done for the DNA fragments isolated from each strain of *Bacillus thuringiensis.* Prehybridization and hybridization of the filter was carried out in a solution of 10X Denhardt's solution [Molecular Cloning, A Laboratory Manual, Sambrook et al. eds. Cold Spring Harbor Press: New York (1989)], 150 g/ml sheared salmon sperm DNA, 1% SDS, 50 mM sodium phosphate pH 7, 5 mM EDTA, 6X SSC, 0.05% sodium pyrophosphate. Prehybridization was at 65 °C for 4 hours and hybridization was at 65 °C for 18 hrs with 1 million cpm/ml of a ³²P-dCTP labeled probe in a volume of 50 ml. Radiolabeled DNA probes were prepared using a BRL random prime labeling system [GIBCO-BRL, Grand Island, New York, USA] and unincorporated counts removed using Nick Columns (Pharmacia).

Filters were probed with a crylB radiolabeled fragment generated by Polymerase Chain Reaction. The generated fragment spans the region from 461-1366 bp of the crylB gene. Hybridization probes were boiled 5 minutes before addition to the hybridization solution. The filters were washed twice in 50 ml of 2X SSC, 0.5% SDS at 65 °C for 20 minutes. The probed and washed filters were exposed to Kodak X-Omat AR X-ray film with Dupont Cronex Lightning Plus intensifying screens at -80 °C. Those colonies which were positive by hybridization were identified and picked from the regrown plates. Picked colonies were streaked on L-agar with 100 µg/ml ampicillin. Plasmid DNA was isolated from each streaked culture using the alkaline miniprep method described in Molecular Cloning, A Laboratory Manual, Sambrook et al. eds. Cold Spring Harbor Press: New York (1989).

Clones designated CGE5618 [carrying the gene for CryIE(c)] and CGE5621 [carrying the gene for CryIE(d)] were isolated. Both clones contained a recombinant plasmid which was shown by the above procedure to possess a DNA fragment isolated from *Bacillus thuringiensis* strain CGB316 (CryIE(c) gene) and CGB323 (CryIE(d) gene) which positively hybridized to the CrylB probe.

The DNA fragment isolated from the appropriate *Bacillus thuringiensis* strains and contained in the recombinant plasmids designated above was re-cloned into pHT3101 [Arantes *et al,* Gene 108, 115-119, 1991], a shuttle vector which allows plasmid manipulation in either *E. coli* or *Bacillus thuringiensis.* pHT3101 is composed of pUC18, a *Bt* replicon and an erythromycin gene for selection in *Bt* (Lereclus et al, FMS Microbiology Letters 60:211-218, 1989). The recombinant plasmid containing the cloned DNA from *Bacillus thuringiensis* was isolated from the *E. coli* cells using standard procedures (Molecular Cloning, A Laboratory Manual, Sambrook et al. eds. Cold Spring Harbor Press: New York (1989)). To remove the cloned *Bacillus thuringiensis* DNA from the recombinant plasmid, the isolated plasmid from each clone was digested with the restriction enzymes *Sac* I and *Bbu* I. The digestion mixtures were then loaded on a 1% SeaPlaque® agarose gel using TBE buffer system and electrophoresed overnight at 25 volts. After electrophoresis, a DNA fragment of approximately 7 kb in size was isolated from the gel using the above described procedures. The DNA fragment isolated from the gel was ligated into pHT31 01 using the previously described procedure by combining 5 µl of the melted (65 °C) agarose fragment and 4 µl 10 ng/µl of pHT3101.

The *Bacillus thuringiensis* DNA isolated from the strain CGB316 and ligated in pHT3101 was designated pCIB5618. The *E. coli* strain containing plasmid pCIB5618 was designated CGE5618. The DNA isolated from the strain CGB323 and ligated in pHT31 01 was designated pCIB5621. The *E*. *coli* strain containing plasmid pCIB5621 was designated CGE5621. Both CGE5621 and CGE5618 were deposited on June 1, 1994 with the Agricultural Research Service, Patent Culture Collection (NRRL), Northern Regional Research Center, 1815 North University Street, Peoria, Illinois 61604, U.S.A. and were assigned accession numbers NRRL B-21276 and NRRL B-21275, respectively.

### Example 2: DNA Sequence Analysis of Novel Toxin Gene and Homology of Deduced Protein Sequence to other Cryl Genes

The DNA sequence of each the novel toxin genes of the present invention was obtained by first synthesizing sequencing primers using an Applied Biosystems model 380B DNA synthesizer. The primers were then used in the DNA sequencing reactions. Sequencing was performed using the dideoxy chain-termination method (Sanger et al., Proc Natl Acad Sci USA 74: 5463-5467, 1977). Sequencing reactions were carried out using the Sequenase system (US Biochemical Corp.) and gel analysis performed on 40 cm gels of 6% polyacrylamide with 7 M urea in Tris-Borate-EDTA buffer (BRL Gel-Mix 6). For CryIE(d), DNA sequencing was obtained from an Applied Biosystem DNA Sequencer, Model 373A, according to the manufacturer's suggested protocol. Analysis of sequences was done using the University of Wisconsin Genetic Computer Group Sequence Analysis Software (UWGCG) and using Gene Codes Corp. (Ann Arbor, Michigan) sequencing program.

According the nomenclature scheme of Höfte and Whiteley, Microbiol. Rev. 53: 242-255 (1989) the proteins encoded by the novel toxin genes would be classified as a CryIE-type, and have been designated herein as CrylE(c) and CrylE(d). Both genes of the present invention encode a crystal protein of 1176 amino acids with a predicted molecular mass of 130.7 kDa. The complete nucleotide sequence of each isolated novel toxin gene is disclosed in SEQ ID NO:1 for CryIE(c) and in SEQ ID NO:7 for CryIE(d). The deduced amino acid sequence of the encoded protein CryIE(c) is disclosed in SEQ ID NO:2 and the encoded protein CryIE(d) is disclosed in SEQ ID NO:8. Using the FASTA program available in UWGCG, the DNA sequence of the two novel toxin genes of the present invention have 99 % sequence identity. Both CryIE(c) and CryIE(d) each have 87% sequence identity to the known endotoxin gene designated CryIE(a) (GenBank/EMBL Accession No. M73252) and 85% sequence identity to the known endotoxin gene CryIE(b) (GenBank/EMBL Accession No. M73252). The deduced protein sequence for both CryIE(c) and CryIE(d) have 83% similarity to CryIE(b) and 81% similarity to CryIE(a).

### Example 3: Transformation of Bacillus thuringiensis with Novel Toxin Genes

The cloned gene for the novel toxin present in the recombinant plasmids pCIB5618 and pCIB5621 was transformed into the acrystalliferous derivative of *Bacillus thuringiensis* designated CGB324. Transformation was accomplished by the method of Schurter et al. (Mol. Gen. Genet. 218: 177-181 (1989)), which is also disclosed in EP-A 0 342 633 which is incorporated herein in its entirety.

Spores of CGB324 were inoculated into 10 ml of L-broth and incubated overnight at 25 °C on a rotary shaker at 100 rpm. After incubation, the culture is diluted 50-fold into L-broth and incubated further at 30 °C on a rotary shaker at 250 rpm until the culture reaches an OD₅₅₀ of 0.2. The bacterial cells are harvested by centrifugation and resuspended in 1/40 volume ice-cold electroporation buffer (400 mM sucrose, 1 mM MgCl, 7 mM phosphate buffer pH 6.0, 20% glycerol). The centrifugation and resuspension of the cells is repeated as described above. Four hundred µl of the resuspended cells are added to the cuvette of a Genepulser with a 0.4 cm electrode gap. Plasmid DNA is added to the cells in the cuvette and maintained at 4 °C for 10 minutes. The plasmid DNA is transferred into the cells by electroporation using a capacitance of 25 F and a field strength of 1300 V. The treated cells were then maintained at 4 °C for an additional 10 minutes, then diluted with 1.6 ml L-broth and incubated for 4 hours at 30 °C on a rotary shaker at 250 rpm. The cells were then plated on T3 agar (3 g Tryptone, 2 g Tryptose, 1.5 g Yeast Extract, 0.05 g MgCl₂, 50 mM sodium phosphate pH 6.8) containing 25 µg/ml erythromycin and incubated at 30 °C for 24-36 hours to visualize colonies. Single colonies were picked from the plates and streaked out on fresh T3 containing erythromycin and grown to sporulation. Transformed cells of CGB324 which produced crystalline protein bodies, indicative of expression of the novel toxin genes, were identified microscopically.

The recombinant strain of *Bacillus thuringiensis* containing pCIB5618 and expressing the novel toxin gene CryIE(c) was designated CGB311. The recombinant strain of *Bacillus thuringiensis* containing pCIB5621 and expressing the novel toixn gene CryIE(d) was designated CGB313. Both CGB313 and CGB311 were deposited on June 1, 1994 with the Agricultural Research Service, Patent Culture Collection (NRRL), Northern Regional Research Center, 1815 North University Street, Peoria, Illinois 61604, U.S.A. and were assigned accession numbers NRRL B-21274 and NRRL B-21273, respectively.

### Example 4: Insect Bioassay of Novel Toxin Genes Expressed in Recombinant Bacillus thuringiensis

Insect bioassays were conducted by adding spore/crystal mixtures to artificial diet preparations. For example, molten Black Cutworm artificial insect diet was poured into 45 mm Gellman snap-cap Petri dishes. Test solutions of spore/crystals mixtures from the recombinant *Bacillus thuringiensis* strain CGB311 and CGB313 were made using dilutions of a 1 mg/ml suspension in 0.01% Triton X-100 designed to provide a range of test concentrations for each sample. After solidification of the molten diet, 100 µl of the appropriate dilution of Bt in 0.01% Triton X-100 was spread over the surface and the plates are air dried. Ten insects at the first instar of *Spodoptera exguia* (beet armyworm), *S*. *fugiperda* (fall armyworm), or *Ostrinia nubilalis* (European corn borer) and which were less than 24 hours old were placed onto the diet surface for a total of 30 insects at the first instar for each concentration of spore/crystal tested. The assay was incubated at 30 °C in complete darkness for 72 hours. The percent mortality was recorded at the end of the assay period.

The assay for *Heliothis virescens* (tobacco budworm) was similar to the above assay with the following differences. Costar 24-well cell culture clusters were used with a single well surface area of 2.26 cm² and 15 µl of the spore/crystal dilution was applied to the surface of the solidified artificial diet. One *H. virescens* larvae which is less than 24 hours old is placed in each well for a total of 24 insects for each concentration of spore/crystal tested. The wells were covered with two pieces of Parafilm and a piece of Teri-Kimwipe® [Fisher Scientific, Pittsburg, Pennsylvania, USA] to prevent escape of the insects from the wells. The assay was incubated at 30 °C for 72 hours after which percent mortality was recorded. Background mortality for all assays was 0-15%.

*Plutella xylostella* bioassays were performed by incorporating aliquots of a 50 mg/ml spore/crystal mixture into molten, artificial *P. xyostella* diet (Biever and Boldt, Annals of Entomological Society of America,1971; Shelton, *et al.*, J. Ent. Sci 26:17) at an appropriate concentration. The 4 ml mixed toxic diet was poured into 1 oz. clear plastic cups (Bioserve product #9051). Subsequent dilutions were mande by adding non-toxic diet to the previous concentration . Once the diet cooled, 5 neonate *P. xyostella* from a diet-adapted lab colony were placed in each diet-containing cup and then covered with a white paper lid (Bioserve product #9049). Twenty larvae were assayed per concentration. Trays of cups were placed in an incubator for 3 days at 72°F with a 14:10 (hours) light:dark cycle. The percent mortality was recorded at the end of the assay period.

The results of the bioassay of the novel toxins are summarized in Table 3, below.

### Table 3: Relative Activities of Novel Toxins on Selected Insects

| **Insect Toxins** | *Spodoptera exigua* | *Spodoptera frugiperda* | *Heliothis virescens* | *Ostrinia nubilalis* | *Plutella xylostella* |
|---|---|---|---|---|---|
| CGB311 containing crylE(c) of the present invention | - | - | + | - | ++ |
| CGB313 containing cryIE(d) of the present invent. | - | ++ | + | na | +++ |
| CryIE(a)^{1/} | ++ | na | - | na | |
| CryIA(a)^{1/} | - | na | ++ | na | - |

| | | | | | |
|---|---|---|---|---|---|
| +++ = 1 ng/cm² ≤ LC₅₀ ≤ 10 ng/cm² | | | | | |
| ++ = 10 ng/cm² ≤ LC₅₀ ≤ 100 ng/cm² | | | | | |
| + = 100 ng/cm² ≤ LC₅₀ ≤ 1000 ng/cm² | | | | | |
| - = LC₅₀ ≥ 1000 ng/cm² | | | | | |
| na = data not available | | | | | |
| ^{1/} = Data taken from Koziel *et al*., In: Biotechnology and Genetic Engineering Reviews, Vol 11, p. 171-228, 1993, Intercept Ltd., Hampshire, UK. | | | | | |

### Example 5: SDS-PAGE Analysis of the Novel Toxin Protein

Spore/crystal stocks of the recombinant strain CGB311 and CGB313 were prepared (20 mg/ml) in 0.1% Triton X-100 from lyophilized powders. A 100 µl portion of the stock was treated with 25 µl of 0.4 N NaOH for 5 min at room temperature, then 125 µl of 4% SDS, 20% glycerol, 10% β-mercapto-ethanol, 0.01% bromophenol blue, and 0.125M Tris-HCL pH 6.8 (Brussock, S.M., and T.C. Currier (1990), In: Analytical chemistry of *B.thuringiensis.* L.A. Hickle and W.L. Fitch, editors, American Chemical Society). This solution was then heated at 100 °C for 2 min. Samples were centrifuged for 60 s at 14,000 g in an Eppendorf 5415 microfuge. The samples were loaded on a 4-12% SDS-PAGE (Novex) and run as described by the supplier. Gels were stained in 0.2% Coomassie blue, 40% methanol and 10% acetic acid for 30 min at room temperature and destained with 40% methanol, 10% acetic acid. Gels were scanned using Molecular Dynamics Personal Densitometer and data was analyzed using Microsoft Excel. Figure 1 shows a protein band at the expected molecular weight based on the deduced protein sequence for each of the novel toxin genes.

### Example 6: Transformation of Maize with the Novel Toxin Gene

Transformation of maize with at least on of the novel toxin genes prepared according to any of the above methods is achieved by microprojectile bombardment of either immature zygotic embryos or serially-propagatable Type I embryogenic callus with a suitable plant transformation vector comprising the said toxin genes.

### 6.1 Construction of Plant Transformation Vectors

Numerous transformation vectors are available for plant transformation, and the genes of this invention can be used in conjunction with any such vectors. The selection of vector for use will depend upon the preferred transformation technique and the target species for transformation. For certain target species, different antibiotic or herbicide selection markers may be preferred. Selection markers used routinely in transformation include the *nptll* gene which confers resistance to kanamycin and related antibiotics (Messing & Vierra, *Gene 19:* 259-268 (1982); Bevan *et al., Nature 304*:184-187 (1983)), the bargene which confers resistance to the herbicide phosphinothricin (White *et al., Nucl Acids Res* 18:1062 (1990), Spencer *et al.* Theor Appl Genet 79: 625-631(1990)), the *hph* gene which confers resistance to the antibiotic hygromycin (Blochinger & Diggelmann, *Mol Cell Biol 4:* 2929-2931), and the *dhfr* gene, which confers resistance to methotrexate (Bourouis *et al., EMBO J. 2(7)*: 1099-1104 (1983)).
(a) Construction of Vectors Suitable for *Agrobacterium* Transformation Many vectors are available for transformation using *Agrobacterium tumefaciens*. These typically carry at least one T-DNA border sequence and include vectors such as pBIN19 (Bevan, *Nucl. Acids Res.* (1984)). Below the construction of two typical vectors is described.

### Construction of pCIB200 and pCIB2001

The binary vectors pCIB200 and pCIB2001 are used for the construction of recombinant vectors for use with *Agrobacterium* and is constructed in the following manner. pTJS75kan is created by *Narl* digestion of pTJS75 (Schmidhauser & Helinski, *J Bacteriol. 164*: 446-455 (1985)) allowing excision of the tetracycline-resistance gene, followed by insertion of an *Accl* fragment from pUC4K carrying an NPTII (Messing & Vierra, *Gene 19:* 259-268 (1982); Bevan *et al., Nature* 304: 184-187 (1983); McBride *et al., Plant Molecular Biology 14*: 266-276 (1990)). *XhoI* linkers are ligated to the *EcoRV* fragment of pCIB7 which contains the left and right T-DNA borders, a plant selectable *nos*/*nptil* chimeric gene and the pUC polylinker (Rothstein *et al., Gene 53*: 153-161 (1987)), and the *XhoI*-digested fragment is cloned into *SalI*-digested pTJS75kan to create pCIB200 (see also EP 0 332 104, example 19 [1338]). pCIB200 contains the following unique polylinker restriction sites: *EcoRI, Sstl, KpnI, BglII, Xbal,* and *Sall. pCIB2001* is a derivative of pCIB200 which created by the insertion into the polylinker of additional restriction sites. Unique restriction sites in the palylinker of pCIB2001 are *EcoRI*, *Sstl*, *KpnI, BgIII, XbaI, SaII, Mlul*, *BclI, AvrII, Apal, Hpal,* and *StuI*. pCIB2001, in addition to containing these unique restriction sites also has plant and bacterial kanamycin selection, left and right T-DNA borders for *Agrobacterium-*mediated transformation, the RK2-derived *trfA* function for mobilization between *E. coli* and other hosts, and the *OriT*and *OriV* functions also from RK2. The pCIB2001 polylinker is suitable for the cloning of plant expression cassettes containing their own regulatory signals.

### Construction of pCIB10 and Hygromycin Selection Derivatives thereof

The binary vector pCIB10 contains a gene encoding kanamycin resistance for selection in plants, T-DNA right and left border sequences and incorporates sequences from the wide host-range plasmid pRK252 allowing it to replicate in both *E. coli* and *Agrobacterium.* Its construction is described by Rothstein *et al*., *Gene 53:* 153-161 (1987). Various derivatives of pCIB10 have been constructed which incorporate the gene for hygromycin B phosphotransferase described by Gritz *et al., Gene 25*: 179-188 (1983)). These derivatives enable selection of transgenic plant cells on hygromycin only (pCIB743), or hygromycin and kanamycin (pCIB715, pCIB717) [Rothstein *et al., Gene 53*: 153-161 (1987)].

### (2) Construction of Vectors Suitable for non-Agrobacterium Transformation.

Transformation without the use of *Agrobacterium tumefaciens* circumvents the requirement for T-DNA sequences in the chosen transformation vector and consequently vectors lacking these sequences can be utilized in addition to vectors such as the ones described above which contain T-DNA sequences. Transformation techniques which do not rely on *Agrobacterium* include transformation via particle bombardment, protoplast uptake (*e.g*. PEG and electroporation) and microinjection. The choice of vector depends largely on the preferred selection for the species being transformed. Below, the construction of some typical vectors is described.

### Construction of pCIB3064

pCIB3064 is a pUC-derived vector suitable for direct gene transfer techniques in combination with selection by the herbicide basta (or phosphinothricin). The plasmid pCIB246 comprises the CaMV 35S promoter in operational fusion to the *E. coli* GUS gene and the CaMV 35S transcriptional terminator and is described in the PCT published application WO 93/07278. The 35S promoter of this vector contains two ATG sequences 5' of the start site. These sites are mutated using standard PCR techniques in such a way as to remove the ATGs and generate the restriction sites *Sspl* and *Pvull.* The new restriction sites are 96 and 37 bp away from the unique *Sall* site and 101 and 42 bp away from the actual start site. The resultant derivative of pCIB246 is designated pCIB3025. The GUS gene is then excised from pCIB3025 by digestion with *Sall* and *Sacl,* the termini rendered blunt and religated to generate plasmid pCIB3060. The plasmid pJIT82 is obtained from the John Innes Centre, Norwich and the a 400 bp *SmaI* fragment containing the *bar* gene from *Streptomyces viridochromogenes* is excised and inserted into the *Hpal* site of pCIB3060 (Thompson *et al*. EMBO J 6: 2519-2523 (1987)). This generated pCIB3064 which comprises the *bar* gene under the control of the CaMV 35S promoter and terminator for herbicide selection, a gene fro ampicillin resistance (for selection in *E. coli*) and a polylinker with the unique sites *Sphl, PstI, Hindlll,* and *BamHI*. This vector is suitable for the cloning of plant expression cassettes containing their own regulatory signals.

### Construction of pSOG19 and pSOG35

pSOG35 is a transformation vector which utilizes the *E*. *coli* gene dihydrofolate reductase (DHFR) as a selectable marker conferring resistance to methotrexate. PCR is used to amplify the 35S promoter (~800 bp), intron 6 from the maize Adh1 gene (~550 bp) [Lou et al, Plant J 3: 393-403, 1993; Dennis et al, Nucl Acids Res 12: 3983-4000, 1984] and 18 bp of the GUS untranslated leader sequence from pSOG10. A 250 bp fragment encoding the *E*. *coli* dihydrofolate reductase type II gene is also amplified by PCR and these two PCR fragments are assembled with a *Sacl-Pstl* fragment from pBI221 (Clontech) which comprised the pUC19 vector backbone and the nopaline synthase terminator. Assembly of these fragments generated pSOG19 which contains the 35S promoter in fusion with the intron 6 sequence, the GUS leader, the DHFR gene and the nopaline synthase terminator. Replacement of the GUS leader in pSOG19 with the leader sequence from Maize Chlorotic Mottle Virus (MCMV) generated the vector pSOG35. pSOG19 and pSOG35 carry the pUC gene for ampicillin resistance and have *Hindlll, Sphl, Pstl* and *EcoRl* sites available for the cloning of foreign sequences.

### pSOG 10

This β-Glucuronidase (GUS) expression vector is derived from plasmid pBI121, purchased from Clonetech Laboratories, Palo Alto, California. Intron 6 of the maize Adh1 gene is amplified by PCR from plasmid pB428, described in Bennetzen et al., Proc. Natl. Acad. Sci, USA 81:4125-4128 (1987), using oligonucleotide primers SON0003 and SON0004.

The PCR reaction product is digested with restriction endonuclease BamHI, cleaving the BamHI site added on the 5' end of each PCR primer. The resulting DNA fragment is purified on an agarose gel and ligated into the BamHI site of pBI121, which is between the CaMV35S promoter and the GUS gene. The ligated DNA is transformed into *E.coli* and clones with the Adh1 intron 6 in the same orientation as the GUS gene are identified by restriction digest.

### pSOG 19

This dihydrofolate reductase (DHFR) expression vector is derived by fusing the 35S promotor and Adh1 intron 6 of pSOG10 to the DHFR gene from plasmid pHCO, described in Bourouis and Jarry, EMBO J. 2: 1099-1104 (1983) The 35S promoter and Adh1 intron 6 are produced by PCR amplification of the fragment from pSOG10 using primers SON0031 and SON0010.

The resulting fragment is digested with restriction endonucleases PstI and BspHI and purified on an agarose gel.

The DHFR coding region is produced by PCR amplification of pHCO using primers SON0016 and SON0017.

The resulting fragment is digested with restriction endonucleases Nsol and Sacl and purified on an agarose gel.

The two fragments described above are ligated into a vector fragment prepared from pBI121 by digestion with restriction endonucleases Pstl and Sacl and purification of the 3kb fragment containing the Nos terminator region and pUC19 region of pBI121 on an agarose gel. This three way ligation fuses the 35S promoter-Adhl intron 6-DHFR gene-Nos terminator in correct order and orientation for functional expression in plants.

### pSOG 30

This GUS expression vector is derived from pSOG 10 by the insertion of the maize chlorotic mottle virus (MCMV) leader, described in Lommel et al., Virology 181: 382-385 (1991), into the 35S-GUS gene non-translated leader by a three way ligation.

Both strands of the 17 bp MCMV capsid protein leader sequence plus appropriate restriction endonuclease sights are synthesized and annealed. The resulting double stranded fragment is degested with BamHI and Ncol and purified on an acrylamide gel.

The GUS gene coding region is amplified by PCR using primers SON0039 and SON0041 and pBI121 as a template.

These primers added an Ncol site to the 5' end of GUS and a Sacl site to the 3' end of GUS. The resulting fragment is digested with restriction endonucleases Ncol and Sacl and purified on an agarose gel.

The GUS gene is removed from the plasmid pSOG 10 by digestion with restriction endonuclease Sacl and partial digestion with restriction endonuclease BamHI. The resulting vector, which has a BamHI site and a Sacl site in which to reinsert a coding region behind the 35S promoter-Adh1 intron 6, is purified on an agarose gel.

The three fragments described above are ligated in a three way ligation to produce a gene fusion with the structure: 35Spromoter-Adh1 intron 6-MCMV leader-GUS-Nos terminator, all in the pUC19 vector backbone.

### pSOG 35

The DHFR selectable marker vector is identical to pSOG19, except that the MCMV leader is inserted in the non-translated leader of the DHFR gene to enhance translation. It is created in two steps. First the GUS coding region in pSOG32, a vector identical to pSOG30 except that it contains a modified Adh promoter rather than 35S, is replaced with DHFR coding region from pSOG19 by excising the GUS with Ncol and Sacl and ligating in the DHFR as an Ncol-Sacl fragment. This resulting in vector pSOG33 which has the gene structure Adh promoter-Adh1 intron 6-MCMV leader-DHFR coding region-Nos terminator, with a BglII site between the promoter and Intron and a SacI site between the coding region and the terminator. The BglII-SacI fragment is isolated by restriction endonuclease digestion and agarose gel purification, and ligated into the BamHI and SacI sites of pSOG30, replacing the Adh1 intronZ6-MCMV leader-GUS coding region of pSOG30 with the Adh1 intron 6-MCMV leader-DHFR coding region of pSOG33.

### 6.2 Construction of Plant Expression Cassettes

Gene sequences intended for expression in transgenic plants are firstly assembled in expression cassettes behind a suitable promoter and upstream of a suitable transcription terminator. These expression cassettes can then be easily transferred to the plant transformation vectors described above in Example 6.1.

### Promoter Selection

The selection of a promoter used in expression cassettes will determine the spatial and temporal expression pattern of the transgene in the transgenic plant. Selected promoters will express transgenes in specific cell types (such as leaf epidermal cells, mesophyll cells, root cortex cells) or in specific tissues or organs (roots, leaves or flowers, for example) and this selection will reflect the desired location of expression of the transgene. Alternatively, the selected promoter may drive expression of the gene under a light-induced or other temporally regulated promoter. A further alternative is that the selected promoter be chemically regulated. This would provide the possibility of inducing expression of the transgene only when desired and caused by treatment with a chemical inducer.

### Transcriptional Terminators

A variety of transcriptional terminators are available for use in expression cassettes. These are responsible for the termination of transcription beyond the transgene and its correct polyadenylation. Appropriate transcriptional terminators and those which are known to function in plants and include the CaMV 35S terminator, the *tml* terminator, the nopaline synthase terminator, the pea *rbcS* E9 terminator. These can be used in both monocotyledons and dicotyledons.

### Sequences for the Enhancement or Regulation of Expression

Numerous sequences have been found to enhance gene expression from within the transcriptional unit and these sequences can be used in conjunction with the genes of this invention to increase their expression in transgenic plants.

Various intron sequences have been shown to enhance expression, particularly in monocotyledonous cells. For example, the introns of the maize *Adh1* gene have been found to significantly enhance the expression of the wild-type gene under its cognate promoter when introduced into maize cells. Intron 1 is found to be particularly effective and enhanced expression in fusion constructs with the chloramphenicol acetyltransferase gene (Callis *et al*., Genes Develop. 1: 1183-1200 (1987)). In the same experimental system, the intron from the maize *bronze 1* gene had a similar effect in enhancing expression (Callis *et al., supra*). Intron sequences have been routinely incorporated into plant transformation vectors, typically within the non-translated leader.

A number of non-translated leader sequences derived from viruses are also known to enhance expression, and these are particularly effective in dicotyledonous cells. Specifically, leader sequences from Tobacco Mosaic Virus (TMV, the "W-sequence"), Maize Chlorotic Mottle Virus (MCMV), and Alfalfa Mosaic Virus (AMV) have been shown to be effective in enhancing expression (*e.g.* Gallie *et al*. *Nucl. Acids Res. 15:* 8693-8711 (1987); Skuzeski *et al*. Plant Molec. Biol. 15: 65-79 (1990))

### Targeting of the Gene Product Within the Cell

Various mechanisms for targeting gene products are known to exist in plants and the sequences controlling the functioning of these mechanisms have been characterized in some detail. For example, the targeting of gene products to the chloroplast is controlled by a signal sequence found at the amino terminal end of various proteins and which is cleaved during chloroplast import yielding the mature protein (*e.g*. Comai *et al.* J. *Biol. Chem. 263:* 15104-15109 (1988)). These signal sequences can be fused to heterologous gene products to effect the import of heterologous products into the chloroplast (van den Broeck *et al*. *Nature 313:* 358-363 (1985)). DNA encoding for appropriate signal sequences can be isolated from the 5' end of the cDNAs encoding the RUBISCO protein, the CAB protein, the EPSP synthase enzyme, the GS2 protein and many other proteins which are known to be chloroplast localized.

Other gene products are localized to other organelles such as the mitochondrion and the peroxisome (*e.g.* Unger *et al. Plant Molec. Biol. 13*: 411-418 (1989)). The cDNAs encoding these products can also be manipulated to effect the targeting of heterologous gene products to these organelles. Examples of such sequences are the nuclear-encoded ATPases and specific aspartate amino transferase isoforms for mitochondria. Targeting to cellular protein bodies has been described by Rogers *et al., Proc. Natl. Acad. Sci. USA 82:* 6512-6516 (1985)).

In addition sequences have been characterized which cause the targeting of gene products to other cell compartments. Amino terminal sequences are responsible for targeting to the ER, the apoplast, and extracellular secretion from aleurone cells (Koehler & Ho, *Plant Cell 2*: 769-783 (1990)). Additionally, amino terminal sequences in conjunction with carboxy terminal sequences are responsible for vacuolar targeting of gene products (Shinshi *et al., Plant Molec. Biol. 14:* 357-368 (1990)).

By the fusion of the appropriate targeting sequences described above to transgene sequences of interest it is possible to direct the transgene product to any organelle or cell compartment. For chloroplast targeting, for example, the chloroplast signal sequence from the RUBISCO gene, the CAB gene, the EPSP synthase gene, or the GS2 gene is fused in frame to the amino terminal ATG of the transgene. The signal sequence selected should include the known cleavage site and the fusion constructed should take into account any amino acids after the cleavage site which are required for cleavage. In some cases this requirement may be fulfilled by the addition of a small number of amino acids between the cleavage site and the transgene ATG or alternatively replacement of some amino acids within the transgene sequence. Fusions constructed for chloroplast import can be tested for efficacy of chloroplast uptake by *in vitro* translation of *in vitro* transcribed constructions followed by *in vitro* chloroplast uptake using techniques described by (Bartlett *et al.* In: Edelmann *et al*. (Eds.) Methods in Chloroplast Molecular Biology, Elsevier. pp 1081-1091 (1982); Wasmann *et al. Mol. Gen. Genet. 205:* 446-453 (1986)). These construction techniques are well known in the art and are equally applicable to mitochondria and peroxisomes. The choice of targeting which may be required for expression of the transgenes will depend on the cellular localization of the precursor required as the starting point for a given pathway. This will usually be cytosolic or chloroplastic, although it may is some cases be mitochondrial or peroxisomal. The products of transgene expression will not normally require targeting to the ER, the apoplast or the vacuole.

The above described mechanisms for cellular targeting can be utilized not only in conjunction with their cognate promoters, but also in conjunction with heterologous promoters so as to effect a specific cell targeting goal under the transcriptional regulation of a promoter which has an expression pattern different to that of the promoter from which the targeting signal derives.

### 6.3. Maize transformation

For transformation using immature zygotic embryos, ears are self-pollinated and immature zygotic embryos are obtained approximately 10 days later. Approximately eight hundred immature zygotic embryos are divided among different target plates containing a medium capable of inducing and supporting the formation of embryogenic callus. The immature zygotic embryos are transferred immediately to the same medium but containing 12% sucrose. After 5 hours, the immature zygotic embryos are bombarded with a plasmid or plasmids using the PDS-1000/He device from BioRad. The plasmid or plasmids comprise a selectable marker such as a gene conferring resistance to phosphinothricin and the novel toxin gene prepared for delivery to and expression in maize according to the above description. The plasmid or plasmids are precipitated onto 1 µm gold particles essentially according to the published procedure from BioRad. The particles are delivered using a burst pressure of 1550 psi of helium. Each target plate is shot twice with the plasmid and gold particle preparation. Since the plasmid or plasmids comprise a chimeric gene coding for resistance to phosphinothricin this substance is used to select transformed cells in vitro. The selection agent is applied at 10 mg/L on the day of gene delivery and increased to 40 mg/L after approximately one month. The embryogenic callus so obtained is regenerated in the presence of the selection agent phosphinothricin. Plants are obtained from the phosphinothricin resistant embryogenic callus lines. The regenerated plants are assayed for resistance to a susceptible insect. All the plants that are resistant to the insect also express the introduced chimeric novel toxin gene as evidenced by the detection of novel toxin protein in the plant using an ELISA assay. Plants resistant to the insect and expressing the introduced novel toxin gene are transformed.

For transformation of maize using Type I embryogenic callus, the callus is obtained from immature zygotic embryos using standard culture techniques. For gene delivery, approximately 300 mg of the Type I callus is prepared by either chopping with a scalpel blade or by subculturing 3-5 days prior to gene delivery. Prior to gene delivery, the prepared callus is placed onto semi-solid culture medium again containing 12% sucrose. After approximately 4 hours, the tissue is bombarded using the PDS-1000/He Biolistic device from BioRad. The plasmid or plasmids comprising a selectable marker such as a gene conferring resistance to phosphinothricin and the novel toxin gene prepared for delivery to and expression in maize according to the above description are precipitated onto 1 µm gold particles using essentially the standard protocol from BioRad. Approximately 16 hours after gene delivery the callus is transferred to standard culture medium containing 2% sucrose and 1 mg/L phosphinothricin. The callus is subcultured on selection for 8 weeks, after which surviving and growing callus is transferred to standard regeneration medium for the production of plants. The regenerated plants are assayed for resistance to a susceptible insect. All the plants that are resistant to the insect also express the introduced chimeric novel toxin gene as evidenced by the detection of novel toxin protein in the plant using an ELISA assay. Plants resistant to the insect and expressing the introduced novel toxin gene are transformed.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Claims

1. An isolated DNA molecule encoding a toxin protein, wherein said toxin protein comprises a sequence selected from the group consisting of Sub-Sequence A (SEQ ID NO:3), Sub-Sequence B (SEQ ID NO:4), Sub-Sequence A' (SEQ ID NO:9) and Sub-Sequence B' (SEQ ID NO:10), which is active against *Heliothis* species but does not exhibit an activity against *Spodoptera exigua*.

2. The isolated DNA molecule of claim 1 wherein said toxin protein comprises both Sub-Sequence A (SEQ ID NO:3) and Sub-Sequence B (SEQ ID NO:4).

3. The isolated DNA molecule of claim 1 wherein said toxin protein comprises both Sub-Sequence A' (SEQ ID NO:9) and Sub-Sequence B' (SEQ ID NO:10).

4. The isolated DNA molecule of claim 1, wherein said toxin protein is CryIE(c) having the amino acid sequence set forth in SEQ ID NO:2.

5. The isolated DNA molecule of claim 1, wherein said toxin protein is CryIE(d) having the amino acid sequence set forth in SEQ ID NO:8.

6. The isolated DNA molecule of claim 4 having the nucleotide sequence set forth in SEQ ID NO:1.

7. The isolated DNA molecule of claim 5 having the nucleotide sequence set forth in SEQ ID NO:7.

8. The isolated DNA molecule of any one of claims 1 to7, wherein said toxin protein is a recombinant protein.

9. The isolated DNA molecule according to any one of claims 1 to 8, which has been optimized for expression in plants

10. The isolated DNA molecule of claim 9 which has been optimized for expression in maize.

11. The isolated DNA molecule of any one of claims 4 to 10 which encodes the toxic core fragment of the toxin protein CryIE(c) and CrylE(d), respectively.

12. An isolated protein molecule comprising a sequence selected from the group consisting of Sub-Sequence A (SED ID NO:3), Sub-Sequence B (SEQ ID NO:4), Sub-Sequence A' (SEQ 10 NO:9) and Sub-Sequence B' (SEQ ID NO:10), which is active against *Heliothis* species but does not exhibit an activity against *Spodoptera exigua.*

13. An isolated protein molecule according to claim 12 comprising both Sub-Sequence A (SEQ ID NO:3) and Sub-Sequence B (SEQ ID NO:4).

14. An isolated protein molecule according to claim 12 comprising both Sub-Sequence A' (SEQ ID NO:9) and Sub-Sequence B' (SEQ ID NO:10).

15. An isolated protein molecule according to claim 12 having the sequence set forth in SEQ ID NO: 2

16. An isolated protein molecule according to claim 12 having the sequence set forth in SEQ ID NO: 8

17. An isolated protein molecule according to any one of claims 12 to 16 which is a recombinant protein.

18. A recombinant DNA molecule comprising at least one of the DNA molecules according to any one of claims 1 to 11, encoding a toxin protein which is active against *Heliothis* species but does not exhibit an activity against *Spodoptera exigua.*

19. A recombinant DNA molecule according to claim 18 comprising a chimeric DNA construct, wherein the DNA molecule is under the control of expression sequences that are operable in microorganisms and/or plants.

20. A recombinant DNA molecule according to claim 19, comprising a chimeric DNA construct, wherein the DNA molecule according to any one of claims 9 or 10 is under the control of expression sequences that are operable in plants.

21. A recombinant microorganism transformed with at least one of the isolated DNA molecules according to claim 19.

22. A recombinant microorganism comprising at least one DNA molecule encoding a toxin protein wherein said toxin protein comprises a sequence selected from the group consisting of Sub-Sequence A (SEQ ID NO:3), Sub-Sequence B (SEQ ID NO:4), Sub-Sequence A' (SEQ ID NO: 9), Sub-Sequence B' (SEQ ID NO: 10) and a combination of Sub-Sequences A (SEQ ID NO:3), B (SEQ ID NO:4), A' (SEQ ID NO: 9), and/or B' (SEQ ID NO: 10), wherein said toxin protein is active against *Heliothis* species.

23. The recombinant microorganism of claims 21 or 22 wherein said recombinant microorganism is selected from the group consisting of members of the genus *Bacillus, Pseudomonas, Caulobacter, Agmellenum, Rhizobium,* and *Clavibacter* or is a baculovirus or yeast.

24. The recombinant microorganism of claim 23 wherein said recombinant microorganism is the nuclear polyhedrosis virus *Autographica californica.*

25. The recombinant microorganism of claim 23, wherein said recombinant microorganism is *Bacillus thuringiensis*.

26. An entomocidal composition comprising the recombinant microorganism of any one of claims 21 to 25 in an insecticidally-effective amount together with a suitable carrier.

27. An entomocidal composition comprising an isolated protein molecule according to any one of claims 12 to 17 in an insecticidally-effective amount together with a suitable carrier.

28. A transformed plant comprising at least one DNA molecule encoding a toxin protein wherein said toxin protein comprises a sequence selected from the group consisting of Sub-Sequence A (SEQ ID NO:3), Sub-Sequence B (SEQ ID NO:4), Sub-Sequence A' (SEQ ID NO: 9), Sub-Sequence B' (SEQ ID NO: 10) and a combination of Sub-Sequences A (SEQ ID NO:3), B (SEQ ID NO:4), A' (SEQ ID NO: 9), and/or B' (SEQ ID NO: 10), wherein said toxin protein is active against *Heliothis* species.

29. A transformed plant comprising the DNA molecule according to any one of claims 1 to 11.

30. A transgenic plant which is transformed with a recombinant DNA molecule according to any one of claims 18 to 20.

31. A transgenic plant according to claim 30 which is transformed with a recombinant DNA molecule according to claim 20.

32. A transformed plant cell comprising the DNA molecule according to any one of claims 1 to 11.

33. A transformed plant cell which is transformed with a recombinant DNA molecule according to any one of claims 18 to 20.

34. A transformed plant cell according to claim 33 which is transformed with a recombinant DNA molecule according to claim 20.

35. A transformed plant cell according to any one of claims 32 to 34 which is a pollen cell or a zygote.

36. The transformed plant or plant cell of any one of claims 28 to 35 wherein said plant and plant cell respectively is selected from the group consisting of maize, wheat, barley, rice, tobacco, cotton, and soybean.

37. The transformed plant or plant cell of claim 36 which is a maize plant or a cell from a maize plant, respectively.

38. A method of obtaining an isolated DNA molecule encoding a toxin protein which comprises a sequence selected from the group consisting of Sub-Sequence A (SEQ ID NO:3), Sub-Sequence B (SEQ ID NO:4), Sub-Sequence A' (SEQ ID NO:9) and Sub-Sequence B' (SEQ ID NO:10), and which is active against *Heliothis* species but does not exhibit an activity against *Spodoptera exigua*, which method comprises
(a) isolating total DNA from *Bacillus thuringiensis* var *kurstaki;*
(b) establishing a genomic DNA library in a suitable host organism:
(c) probing the said library with a probe molecule comprising DNA according to any one of claims 1 to 11;
(d) isolating a clone that hybridizes with the probe molecule; and
(e) probing the said clone for its insecticidal activity.

39. A method of producing an isolated protein molecule which comprises a sequence selected from the group consisting of Sub-Sequence A (SEQ ID NO:3), Sub-Sequence B (SEQ ID NO:4), Sub-Sequence A' (SEQ ID NO:9) and Sub-Sequence B' (SEQ ID NO:10), and which is active against *Heliothis* species but does not exhibit an activity against *Spodoptera exigua,* which method comprises
(a) transforming a suitable host organism with a recombinant DNA molecule according to any one of claims 18 to 20.
(b) growing the so transformed host organism in a suitable medium; and
(c) isolating the recombinant protein product produced by the transformed host organism upon expression of the toxin gene.

40. A method of producing a recombinant microorganism comprising transforming the said microorganism with a recombinant DNA molecule according to any one of claims 18 or 19.

41. A method of producing a transgenic plant or plant cell according to any one of claims 28 to 37 comprising transforming the said plant and plant cell, respectively, with a recombinant DNA molecule according to any one of claims 18 to 20.

42. A method of producing an entomocidal composition according to any one of claims 26 or 27 comprising mixing the recombinant microorganism of any one of claims 21 to 25 or an isolated protein molecule according to any one of claims 12 to 17 in an insecticidally-effective amount with a suitable carrier.

43. A method of protecting plants against damage caused by an insect pest comprising applying to the plant or the growing area of the said plant an entomocidal composition according to any one of claims 26 or 27.

44. A method of protecting plants against damage caused by an insect pest comprising applying to the plant a toxin protein according to any one of claims 12 to 17.

45. A method of protecting plants against damage caused by an insect pest comprising planting a plant according to any one of claims 28 to 31, 36 and 37 within an area where the said insect pest may occur.

46. Use of an entomocidal composition according to claim 26 or 27 for protecting plants against damage caused by an insect pest.

## Patentansprüche

1. Isoliertes DNA-Molekül, das ein Toxinprotein codiert, worin das Toxinprotein eine Sequenz umfaßt, die aus der Gruppe ausgewählt ist, die aus Untersequenz A (SEQ ID NO:3), Untersequenz B (SEQ ID NO:4), Untersequenz A' (SEQ ID NO:9) und Untersequenz B' (SEQ ID NO:10) besteht, und aktiv gegen Heliothis-Arten ist, aber keine Aktivität gegen Spodoptera exigua aufweist.

2. Isoliertes DNA-Molekül gemäß Anspruch 1, worin das Toxinprotein sowohl Untersequenz A (SEQ ID NO:3) als auch Untersequenz B (SEQ ID NO:4) umfaßt.

3. Isoliertes DNA-Molekül gemäß Anspruch 1, worin das Toxinprotein sowohl Untersequenz A' (SEQ ID NO:9) als auch Untersequenz B' (SEQ ID NO:10) umfaßt.

4. Isoliertes DNA-Molekül gemäß Anspruch 1, worin das Toxinprotein CryIE(c) mit der in SEQ ID NO:2 dargestellten Aminosäuresequenz ist.

5. Isoliertes DNA-Molekül gemäß Anspruch 1, worin das Toxinprotein CryIE(d) mit der in SEQ ID NO:8 dargestellten Aminosäuresequenz ist.

6. Isoliertes DNA-Molekül gemäß Anspruch 4 mit der in SEQ ID NO:1 dargestellten Nukleotidsequenz.

7. Isoliertes DNA-Molekül gemäß Anspruch 5 mit der in SEQ ID NO:7 dargestellten Nukleotidsequenz.

8. Isoliertes DNA-Molekül gemäß einem der Ansprüche 1 bis 7, worin das Toxinprotein ein rekombinantes Protein ist.

9. Isoliertes DNA-Molekül gemäß einem der Ansprüche 1 bis 8, das zur Expression in Pflanzen optimiert wurde.

10. Isoliertes DNA-Molekül gemäß Anspruch 9, das zur Expression in Mais optimiert wurde.

11. Isoliertes DNA-Molekül gemäß einem der Ansprüche 4 bis 10, das das toxische Kernfragment des Toxinproteins CryIE(c) bzw. CryIE(d) codiert.

12. Isoliertes Proteinmolekül, das eine Sequenz umfaßt, die aus der Gruppe ausgewählt ist, die aus Untersequenz A (SEQ ID NO:3), Untersequenz B (SEQ ID NO:4), Untersequenz A' (SEQ ID NO:9) und Untersequenz B' (SEQ ID NO:10) besteht, und das aktiv gegen Heliothis-Arten ist, aber keine Aktivität gegen Spodoptera exigua aufweist.

13. Isoliertes Proteinmolekül gemäß Anspruch 12, das sowohl Untersequenz A (SEQ ID NO:3) als auch Untersequenz B (SEQ ID NO:4) umfaßt.

14. Isoliertes Proteinmolekül gemäß Anspruch 12, das sowohl Untersequenz A' (SEQ ID NO:9) als auch Untersequenz B' (SEQ ID NO:10) umfaßt.

15. Isoliertes Proteinmolekül gemäß Anspruch 12 mit der in SEQ ID NO:2 dargestellten Sequenz.

16. Isoliertes Proteinmolekül gemäß Anspruch 12 mit der in SEQ ID NO:8 dargestellten Sequenz.

17. Isoliertes Proteinmolekül gemäß einem der Ansprüche 12 bis 16, das ein rekombinantes Protein ist.

18. Rekombinantes DNA-Molekül, das wenigstens eines der DNA-Moleküle gemäß einem der Ansprüche 1 bis 11 umfaßt und ein Toxinprotein codiert, das aktiv gegen Heliothis-Arten ist, aber keine Aktivität gegen Spodoptera exigua aufweist.

19. Rekombinantes DNA-Molekül gemäß Anspruch 18, das ein chimäres DNA-Konstrukt umfaßt, worin das DNA-Molekül unter der Kontrolle von Expressionssequenzen ist, die in Mikroorganismen und/oder Pflanzen funktionsfähig sind.

20. Rekombinantes DNA-Molekül gemäß Anspruch 19, das ein chimäres DNA-Konstrukt umfaßt, worin das DNA-Molekül gemäß einem der Ansprüche 9 oder 10 unter der Kontrolle von Expressionssequenzen ist, die in Pflanzen funktionsfähig sind.

21. Rekombinanter Mikroorganismus, der mit wenigstens einem der isolierten DNA-Moleküle gemäß Anspruch 19 transformiert ist.

22. Rekombinanter Mikroorganismus, der wenigstens ein DNA-Molekül umfaßt, das ein Toxinprotein codiert, worin das Toxinprotein eine Sequenz umfaßt, die aus der Gruppe ausgewählt ist, die aus Untersequenz A (SEQ ID NO:3), Untersequenz B (SEQ ID NO:4), Untersequenz A' (SEQ ID NO:9), Untersequenz B' (SEQ ID NO:10) und einer Kombination aus Untersequenzen A (SEQ ID NO:3), B (SEQ ID NO:4), A' (SEQ ID NO:9) und/oder B' (SEQ ID NO:10) besteht, worin das Toxinprotein aktiv gegen Heliothis-Arten ist.

23. Rekombinanter Mikroorganismus gemäß Ansprüchen 21 oder 22, worin der rekombinante Mikroorganismus aus der Gruppe ausgewählt ist, die aus Mitgliedern der Gattung Bacillus, Pseudomonas, Caulobacter, Agmellenum, Rhizobium und Clavibacter besteht, oder.ein Baculovirus oder eine Hefe ist.

24. Rekombinanter Mikroorganismus gemäß Anspruch 23, worin der rekombinante Mikroorganismus das Kernpolyedervirus Autographica californica ist.

25. Rekombinanter Mikroorganismus gemäß Anspruch 23, worin der rekombinante Mikroorganismus Bacillus thuringiensis ist.

26. Insektizide Zusammensetzung, die den rekombinanten Mikroorganismus gemäß einem der Ansprüche 21 bis 25 in einer insektizid wirksamen Menge zusammen mit einem geeigneten Träger umfaßt.

27. Insektizide Zusammensetzung, die ein isoliertes Proteinmolekül gemäß einem der Ansprüche 12 bis 17 in einer insektizid wirksamen Menge zusammen mit einem geeigneten Träger umfaßt.

28. Transformierte Pflanze, die wenigstens ein DNA-Molekül umfaßt, das ein Toxinprotein codiert, worin das Toxinprotein eine Sequenz umfaßt, die aus der Gruppe ausgewählt ist, die aus Untersequenz A (SEQ ID NO:3), Untersequenz B (SEQ ID NO:4), Untersequenz A' (SEQ ID NO:9), Untersequenz B' (SEQ ID NO:10) und einer Kombination aus Untersequenzen A (SEQ ID NO:3), B (SEQ ID NO:4), A' (SEQ ID NO:9) und/oder B' (SEQ ID NO:10) besteht, worin das Toxinprotein aktiv gegen Heliothis-Arten ist.

29. Transformierte Pflanze, die das DNA-Molekül gemäß einem der Ansprüche 1 bis 11 umfaßt.

30. Transgene Pflanze, die mit einem rekombinanten DNA-Molekül gemäß einem der Ansprüche 18 bis 20 transformiert ist.

31. Transgene Pflanzen gemäß Anspruch 30, die mit einem rekombinanten DNA-Molekül gemäß Anspruch 20 transformiert ist.

32. Transformierte Pflanzenzelle, die das DNA-Molekül gemäß einem der Ansprüche 1 bis 11 umfaßt.

33. Transformierte Pflanzenzelle, die mit einem rekombinanten DNA-Molekül gemäß einem der Ansprüche 18 bis 20 transformiert ist.

34. Transformierte Pflanzenzelle gemäß Anspruch 33, die mit einem rekombinanten DNA-Molekül gemäß Anspruch 20 transformiert ist.

35. Transformierte Pflanzenzelle gemäß einem der Ansprüche 32 bis 34, die eine Pollenzelle oder eine Zygote ist.

36. Transformierte Pflanze oder Pflanzenzelle gemäß einem der Ansprüche 28 bis 35, worin die Pflanze oder Pflanzenzelle aus der Gruppe ausgewählt ist, die aus Mais, Weizen, Gerste, Reis, Tabak, Baumwolle und Sojabohne besteht.

37. Transformierte Pflanze oder Pflanzenzelle gemäß Anspruch 36, die eine Maispflanze oder eine Zelle aus einer Maispflanze ist.

38. Verfahren zum Erhalt eines isolierten DNA-Moleküls, das ein Toxinprotein codiert, das eine Sequenz umfaßt, die aus der Gruppe ausgewählt ist, die aus Untersequenz A (SEQ ID NO:3), Untersequenz B (SEQ ID NO:4), Untersequenz A' (SEQ ID NO:9) und Untersequenz B' (SEQ ID NO:10) besteht, und das aktiv gegen. Heliothis-Arten ist, aber keine Aktivität gegen Spodoptera exigua aufweist, wobei das Verfahren die folgenden Schritte umfaßt:
(a) Isolieren von Gesamt-DNA aus Bacillus thuringiensis var. kurstaki;
(b) Einrichten einer genomischen DNA-Bibliothek in einem geeigneten Wirtsorganismus;
(c) Sondieren der Bibliothek mit einem Sondenmolekül, das DNA gemäß einem der Ansprüche 1 bis 11 umfaßt;
(d) Isolieren eines Klons, der mit dem Sondenmolekül hybridisiert; und
(e) Sondieren des Klons auf seine insektizide Aktivität.

39. Verfahren zur Herstellung eines isolierten Proteinmoleküls, das eine Sequenz umfaßt, die aus der Gruppe ausgewählt ist, die aus Untersequenz A (SEQ ID NO:3), Untersequenz B (SEQ ID NO:4), Untersequenz A' (SEQ ID NO:9) und Untersequenz B' (SEQ ID NO:10) besteht, und das aktiv gegen Heliothis-Arten ist, aber keine Aktivität gegen Spodoptera exigua aufweist, wobei das Verfahren die folgenden Schritte umfaßt:
(a) Transformieren eines geeigneten Wirtsorganismus mit einem rekombinanten DNA-Molekül gemäß einem der Ansprüche 18 bis 20;
(b) Kultivieren des so transformierten Wirtsorganismus in einem geeigneten Medium; und
(c) Isolieren des rekombinanten Proteinprodukts, das durch den transformierten Wirtsorganismus bei Expression des Toxingens hergestellt wird.

40. Verfahren zur Herstellung eines rekombinanten Mikroorganismus, das das Transformieren des Mikroorganismus mit einem rekombinanten DNA-Molekül gemäß einem der Ansprüche 18 oder 19 umfaßt.

41. Verfahren zur Herstellung einer transgenen Pflanze oder Pflanzenzelle gemäß einem der Ansprüche 28 bis 37, das das Transformieren der Pflanze oder Pflanzenzelle mit einem rekombinanten DNA-Molekül gemäß einem der Ansprüche 18 bis 20 umfaßt.

42. Verfahren zur Herstellung einer insektiziden Zusammensetzung gemäß einem der Ansprüche 26 oder 27, das das Vermischen des rekombinanten Mikroorganismus gemäß einem der Ansprüche 21 bis 25 oder eines isolierten Proteinmoleküls gemäß einem der Ansprüche 12 bis 17 in einer insektizid wirksamen Menge mit einem geeigneten Träger umfaßt.

43. Verfahren zum Schützen von Pflanzen gegen Schädigung, die durch einen Insektenschädling verursacht wird, das das Ausbringen einer insektiziden Zusammensetzung gemäß einem der Ansprüche 26 oder 27 auf die Pflanze oder das Anbaugebiet der Pflanze umfaßt.

44. Verfahren zum Schützen von Pflanzen gegen Schädigung, die durch einen Insektenschädling verursacht wird, das das Ausbringen eines Toxinproteins gemäß einem der Ansprüche 12 bis 17 auf die Pflanze umfaßt.

45. Verfahren zum Schützen von Pflanzen gegen Schädigung, die durch einen Insektenschädling verursacht wird, das das Pflanzen einer Pflanze gemäß einem der Ansprüche 28 bis 31, 36 und 37 innerhalb eines Gebiets umfaßt, in dem der Insektenschädling auftreten kann.

46. Verwendung einer insektiziden Zusammensetzung gemäß Anspruch 26 oder 27 zum Schützen von Pflanzen gegen Schädigung, die durch einen Insektenschädling verursacht wird.

## Revendications

1. Molécule d'ADN isolée codant pour une protéine toxine, ladite protéine toxine comprenant une séquence choisie dans le groupe constitué par la sous-séquence A (SEQ ID NO. 3), la sous-séquence B (SEQ ID NO. 4), la sous-séquence A' (SEQ ID NO. 9) et la sous-séquence B' (SEQ ID NO. 10), qui est active contre les espèces de *Heliothis,* mais ne présente pas d'activité contre *Spodoptera exigua*.

2. Molécule d'ADN isolée selon la revendication 1, ladite protéine toxine comprenant à la fois la sous-séquence A (SEQ ID NO. 3) et la sous-séquence B (SEQ ID NO. 4).

3. Molécule d'ADN isolée selon la revendication 1, ladite protéine toxine comprenant à la fois la sous-séquence A' (SEQ ID NO. 9) et la sous-séquence B' (SEQ ID NO. 10).

4. Molécule d'ADN isolée selon la revendication 1, ladite protéine toxine étant CryIE(c) ayant la séquence d'aminoacides représentée par SEQ ID NO. 2.

5. Molécule d'ADN isolée selon la revendication 1, ladite protéine toxine étant CryIE(d) ayant la séquence d'aminoacides représentée par SEQ ID NO. 8.

6. Molécule d'ADN isolée selon la revendication 4, ayant la séquence de nucléotides représentée par SEQ ID NO. 1.

7. Molécule d'ADN isolée selon la revendication 5, ayant la séquence de nucléotides représentée par SEQ ID NO. 7.

8. Molécule d'ADN isolée selon l'une quelconque des revendications 1 à 7, ladite protéine toxine étant une protéine recombinée.

9. Molécule d'ADN isolée selon l'une quelconque des revendications 1 à 8, qui a été optimisée pour une expression dans des plantes.

10. Molécule d'ADN isolée selon la revendication 9, qui a été optimisée pour une expression dans le maïs.

11. Molécule d'ADN isolée selon l'une quelconque des revendications 4 à 10, qui code respectivement pour le fragment de noyau toxique de la protéine toxine CryIE(c) et CryIE(d).

12. Molécule de protéine isolée comprenant une séquence choisie dans le groupe constitué par la sous-séquence A (SEQ ID NO. 3), la sous-séquence B (SEQ ID NO. 4), la sous-séquence A' (SEQ ID NO. 9) et la sous-séquence B' (SEQ ID NO. 10), qui est active contre les espèces de *Heliothis,* mais ne présente pas d'activité contre *Spodoptera exigua*.

13. Molécule de protéine isolée selon la revendication 12, comprenant à la fois la sous-séquence A (SEQ ID NO. 3) et la sous-séquence B (SEQ ID NO. 4).

14. Molécule de protéine isolée selon la revendication 12, comprenant à la fois la sous-séquence A' (SEQ ID NO. 9) et la sous-séquence B' (SEQ ID NO. 10).

15. Molécule de protéine isolée selon la revendication 12, ayant la séquence représentée par SEQ ID NO. 2.

16. Molécule de protéine isolée selon la revendication 12, ayant la séquence représentée par SEQ ID NO. 8.

17. Molécule de protéine isolée selon l'une quelconque des revendications 12 à 16, qui est une protéine recombinée.

18. Molécule d'ADN recombiné comprenant au moins l'une des molécules d'ADN selon l'une quelconque des revendications 1 à 11, codant pour une protéine toxine qui est active contre les espèces de *Heliothis,* mais ne présente pas d'activité contre *Spodoptera exigua*.

19. Molécule d'ADN recombiné selon la revendication 18, comprenant un assemblage d'ADN chimère, dans lequel la molécule d'ADN est sous le contrôle de séquences d'expression qui sont fonctionnelles dans des micro-organismes et/ou des plantes.

20. Molécule d'ADN recombiné selon la revendication 19, comprenant un assemblage d'ADN chimère, dans lequel la molécule d'ADN selon l'une quelconque des revendications 9 ou 10 est sous le contrôle de séquences d'expression qui sont fonctionnelles dans des plantes.

21. Micro-organisme recombiné transformé avec au moins l'une des molécules d'ADN isolées selon la revendication 19.

22. Micro-organisme recombiné comprenant au moins une molécule d'ADN codant pour une protéine toxine, ladite protéine toxine comprenant une séquence choisie dans le groupe constitué par la sous-séquence A (SEQ ID NO. 3), la sous-séquence B (SEQ ID NO. 4), la sous-séquence A' (SEQ ID NO. 9), la sous-séquence B' (SEQ ID NO. 10) et une combinaison de sous-séquences A (SEQ ID NO. 3), B (SEQ ID NO. 4), A' (SEQ ID NO. 9) et/ou B' (SEQ ID NO. 10), ladite protéine toxine étant active contre les espèces de *Heliothis*.

23. Micro-organisme recombiné selon les revendications 21 ou 22, ledit micro-organisme recombiné étant choisi dans le groupe constitué par des membres du genre *Bacillus, Pseudomonas, Caulobacter, Agmellenum, Rhizobium* et *Clavibacter,* ou étant un baculovirus ou une levure.

24. Micro-organisme recombiné selon la revendication 23, ledit micro-organisme recombiné étant le virus de la polyédrose nucléaire *Autographica californica*.

25. Micro-organisme recombiné selon la revendication 23, ledit micro-organisme recombiné étant *Bacillus thuringiensis.*

26. Composition insecticide comprenant le micro-organisme recombiné selon l'une quelconque des revendications 21 à 25 en une quantité efficace comme insecticide avec un support approprié.

27. Composition insecticide comprenant une molécule de protéine isolée selon l'une quelconque des revendications 12 à 17 en une quantité efficace comme insecticide avec un support approprié.

28. Plante transformée comprenant au moins une molécule d'ADN codant pour une protéine toxine, ladite protéine toxine comprenant une séquence choisie dans le groupe constitué par la sous-séquence A (SEQ ID NO. 3), la sous-séquence B (SEQ ID NO. 4), la sous-séquence A' (SEQ ID NO. 9), la sous-séquence B' (SEQ ID NO. 10), et une combinaison de sous-séquences A (SEQ ID NO. 3), B (SEQ ID NO. 4), A' (SEQ ID NO. 9) et/ou B' (SEQ ID NO. 10), ladite protéine toxine étant active contre les espèces de *Heliothis*.

29. Plante transformée comprenant la molécule d'ADN selon l'une quelconque des revendications 1 à 11.

30. Plante transgénique qui est transformée avec une molécule d'ADN recombiné selon l'une quelconque des revendications 18 à 20.

31. Plante transgénique selon la revendication 30, qui est transformée avec une molécule d'ADN selon la revendication 20.

32. Cellule de plante transformée comprenant la molécule d'ADN selon l'une quelconque des revendications 1 à 11.

33. Cellule de plante transformée, qui est transformée avec une molécule d'ADN recombiné selon l'une quelconque des revendications 18 à 20.

34. Cellule de plante transformée selon la revendication 33, qui est transformée avec une molécule d'ADN recombiné selon la revendication 20.

35. Cellule de plante transformée selon l'une quelconque des revendications 32 à 34, qui une cellule de pollen ou un zygote.

36. Plante ou cellule de plante transformée selon l'une quelconque des revendications 28 à 35, lesdites plante et cellule de plante étant choisies respectivement dans le groupe constitué par le maïs, le blé, l'orge, le riz, le tabac, le coton et le soja.

37. Plante ou cellule de plante transformée selon la revendication 36, qui est respectivement un plant de maïs ou une cellule d'un plant de maïs.

38. Procédé d'obtention d'une molécule d'ADN isolée codant pour une protéine toxine qui comprend une séquence choisie dans le groupe constitué par la sous-séquence A (SEQ ID NO. 3), la sous-séquence B (SEQ ID NO. 4), la sous-séquence A' (SEQ ID NO. 9) et la sous-séquence B' (SEQ ID NO. 10), qui est active contre les espèces de *Heliothis*, mais ne présente pas d'activité contre *Spodoptera exigua*, ce procédé comprenant:
(a) l'isolement de l'ADN total de *Bacillus thuringiensis* var. *kurstaki*;
(b) l'établissement d'une banque d'ADN génomique dans un organisme hôte approprié;
(c) le criblage de ladite banque avec une molécule sonde comprenant un ADN selon l'une quelconque des revendications 1 à 11;
(d) l'isolement d'un clone qui s'hybride à la molécule sonde; et
(e) le criblage dudit clone pour son activité insecticide.

39. Procédé de production d'une molécule de protéine isolée qui comprend une séquence choisie dans le groupe constitué par la sous-séquence A (SEQ ID NO. 3), la sous-séquence B (SEQ ID NO. 4), la sous-séquence A' (SEQ ID NO. 9) et la sous-séquence B' (SEQ ID NO. 10), et qui est active contre les espèces de *Heliothis*, mais ne présente pas d'activité contre *Spodoptera exigua*, ce procédé comprenant:
(a) la transformation d'un organisme hôte approprié avec une molécule d'ADN recombiné selon l'une quelconque des revendications 18 à 20;
(b) la culture de l'organisme hôte ainsi transformé dans un milieu approprié; et
(c) l'isolement du produit protéinique recombiné produit par l'organisme hôte transformé par expression du gène de la toxine.

40. Procédé de production d'un micro-organisme recombiné comprenant la transformation dudit micro-organisme avec une molécule d'ADN recombiné selon l'une quelconque des revendications 18 ou 19.

41. Procédé de production d'une plante ou d'une cellule de plante transgénique selon l'une quelconque des revendications 28 à 37, comprenant la transformation respective desdites plante et cellule de plante avec une molécule d'ADN recombiné selon l'une quelconque des revendications 18 à 20.

42. Procédé de production d'une composition insecticide selon l'une quelconque des revendications 26 ou 27, comprenant le mélange du micro-organisme recombiné selon l'une quelconque des revendications 21 à 25 ou d'une molécule de protéine isolée selon l'une quelconque des revendications 12 à 17 en une quantité efficace comme insecticide avec un support approprié.

43. Procédé de protection de plantes contre les dommages entraînés par un insecte nuisible, comprenant l'application à la plante ou à la zone de culture de ladite plante d'une composition insecticide selon l'une quelconque des revendications 26 ou 27.

44. Procédé de protection de plantes contre les dommages entraînés par un insecte nuisible, comprenant l'application à la plante d'une protéine toxine selon l'une quelconque des revendications 12 à 17.

45. Procédé de protection de plantes contre les dommages entraînés par un insecte nuisible, comprenant la plantation d'une plante selon l'une quelconque des revendications 28 à 31, 36 et 37 dans une zone dans laquelle ledit insecte nuisible peut se rencontrer.

46. Utilisation d'une composition insecticide selon la revendication 26 ou 27 pour la protection de plantes contre les dommages entraînés par un insecte nuisible.
